# EUROPÄISCHE PATENTSCHRIFT

(11) **EP 1 613 584 B1**
(45) Veröffentlichungstag und Bekanntmachung des Hinweises auf die Patenterteilung: **21.11.2007**
(21) Anmeldenummer: 04725610.2
(22) Anmeldetag: 03.04.2004
(51) Int. Cl.: C07C 217/62, A61K 31/135, C07C 213/10, A61P 13/00

(54) **PHARMAZEUTISCHE FORMULIERUNGEN ENTHALTEND HOCHREINE BASEN VON 3,3-DIPHENYLPROPYLAMINMONOESTERN**
PHARMACEUTICAL FORMULATIONS COMPRISING HIGHLY PURE BASES OF 3,3-DIPHENYL PROPYLAMINE MONOESTERS
PREPARATIONS PHARMACEUTIQUES COMPRENANT DES BASES DE GRANDE PURETE DE MONOESTERS DE LA 3,3-DIPHENYLPROPYLAMINE

(30) Priorität: 08.04.2003 DE 10315917
(43) Veröffentlichungstag der Anmeldung: 11.01.2006
(73) Patentinhaber: SCHWARZ PHARMA AG, 40789 Monheim (DE)
(72) Erfinder: BREITENBACH, Armin, 51371 Leverkusen (DE); MEESE, Claus, 40789 Monheim (DE); WOLFF, Hans-Michael, 40789 Monheim (DE); DREWS, Roland, 40789 Monheim (DE)
(86) Internationale Anmeldenummer: PCT/EP2004/003567
(87) Internationale Veröffentlichungsnummer: WO 2004/089872

(56) Entgegenhaltungen:
- WO-A-00/12070
- WO-A-01/35957
- WO-A-94/11337
- WO-A-99/58478

## Beschreibung

Die vorliegende Erfindung betrifft hochreine Basen von 3,3-Diphenylpropylaminmonoestern, ihre Herstellung und Verwendung als Arzneimittel, insbesondere zur transdermalen und transmukosalen Administration.

In den vergangenen 50 Jahren ist der Anteil der Betagten innerhalb der Gesamtbevölkerung erheblich gestiegen. In dieser Gruppe gehören Blasenfunktionsstörungen zu den häufigsten Alterskrankheiten. Daher kommt der Entwicklung einer möglichst effektiven und schonenden Therapie von Blasenerkrankungen eine immer größere und besondere Bedeutung zu.

Bei der Dranginkontinenz liegt die Störung in einer Fehlfunktion des Blasenmuskels. Die Ursache ist dabei häufig eine Stimulation bzw. Hyperaktivität der muskarinergen Rezeptoren. Aus diesem Grund werden zur Therapie der hyperaktiven Blase und der damit verbundenen Symptome wie erhöhter Harndrang, Inkontinenz, Pollakisurie oder Nykturie bevorzugt die antimuskarinergen Wirkstoffe Tolterodin und Oxybutynin eingesetzt.

Oxybutynin ist ein effektiver antimuskarinerger Wirkstoff, der jedoch beträchtliche Nebenwirkungen hat. Insbesondere wird von vielen Patienten die ausgeprägte Mundtrockenheit als äußerst unangenehm empfunden.

Tolterodin scheint gegenüber Oxybutynin den Vorteil einer niedrigeren muskarinergen Nebenwirkungsrate aufzuweisen. Tolterodin wird im Organismus vorwiegend über das Cytochrom P 450-lsoenzym 2D6 zum aktiven Hauptmetaboliten 2-[3-(1,1-Diisopropylamino)-1-phenylpropyl]-4-(hydroxymethyl)phenol sowie - langsam - durch das Cytochrom P 450 Isoenzym 3A4 zum inaktiven Metaboliten dealkyliert.

Da Tolterodin ausschließlich über P450-lsoenzyme metabolisiert wird, besteht die potentielle Gefahr der Interaktionen mit dem Abbau anderer Wirkstoffe, z.B. mit Warfarin (Colucci, Annals of Pharmacotherapy 33, 1999, 1173), Antimykotika wie Ketoconazol (Brynne, Br J Clin Pharmacol 48, 1999, 564), Makrolidantibiotika, oder Proteasehemmern. Diese Gefahr besteht insbesondere bei sogenannten Langsam-Metabolisierern, die einen Mangel an 2D6 haben, Tolterodin ausschließlich über 3A4 metabolisieren und eine deutlich erhöhte Tolterodin-Konzentration im Plasma aufweisen.

Die WO 99/58 478 beschreibt neue Derivate von 3,3-Diphenylpropylaminen als muskarinerge Wirkstoffe. Die offenbarten 3,3-Diphenylpropylamin-Derivate sind Prodrugs von 2-[3-(1,1-Diisopropylamino)-1-phenylpropyl]-4-(hydroxymethyl)phenol und werden beim Durchtritt durch biologische Membranen sowie im Plasma durch Esterasen hydrolysiert. Damit entfällt der 2D6-abhängige Abbaumechanismus.

Solche 3,3-Diphenylpropylamin-Derivate, z.B. 2-[3-(1,1-Diisopropylamino)-1-phenylpropyl]-4-(hydroxymethyl)phenyl isobutyrat (INN: Fesoterodin), neigen daher im Gegensatz zu Tolterodin auch bei Langsam-Metabolisierem nicht zur Akkumulation, interferieren nicht mit P450 Induktoren/Inhibitoren und besitzen im Hinblick auf potentielle Wirkstoffinteraktionen und Wirkstoffakkumulation ein vorteilhaftes Sicherheitsprofil.

Es bestand daher der Bedarf, dem Patientenkollektiv die Vorteile der in WO 99/58478 beschriebenen 3,3-Diphenylpropylamin-Derivate, insbesondere die Vorteile des Fesoterodines, zur Verfügung zu stellen. Gerade der Metabolisierungsweg von Tolterodin und die Nachteile von Oxybutynin (Mundtrockenheit) verdeutlichen den medizinischen Bedarf für ein Arzneimittel, das die Nachteile der beiden vorgenannten Substanzen nicht aufweist.

Die in WO 99/58478 offenbarten Basen der 3,3-Diphenylpropylamine werden hergestellt, indem 2-(3-Diisopropylamino-1-phenylpropyl)-4-hydroxymethylphenol unter basischen Bedingungen mit einem geeigneten Säurechlorid, z.B. lsobuttersäurechlorid, umgesetzt wird (siehe Ausführungsbeispiel 3aa der WO 99/58478).

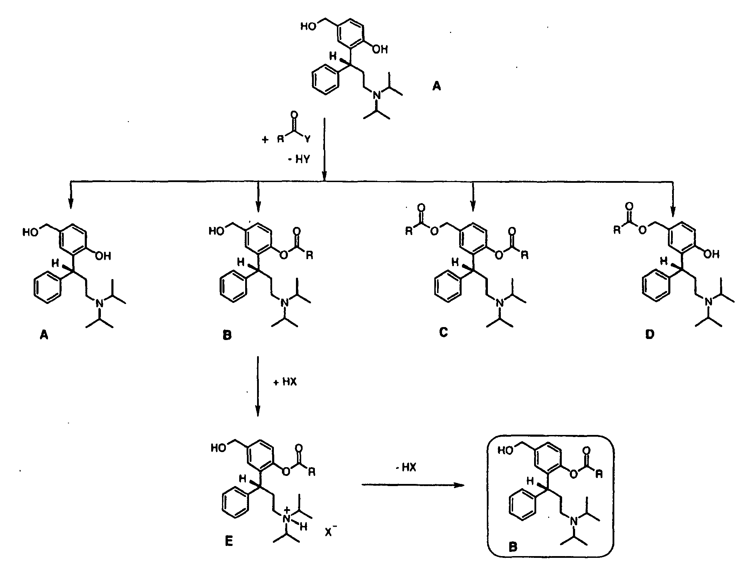

Ungünstigerweise führt diese Reaktion jedoch nur zu ca 90% bis maximal ca. 94% des gewünschten Hauptproduktes (B). Das Produkt enthält regelmäßig 6-10% Verunreinigungen der Ausgangssubstanz (A), des eingesetzten Acylierungsreganzes sowie ungewünschte Reaktionsprodukte in Form des entsprechenden Diesters (C), des Monoesters (D) der 4-Hydroxygruppe (siehe Abbildung 1) sowie durch Dimerisierung/Polymerisation.

Versuche der Erfinder der vorliegenden Patentanmeldung, die Synthese-Reaktion selektiver zu machen, indem z.B. die Menge des Acylierungsreagenz variiert wurde und/oder die Acylierungsbedingungen (Temperatur, Lösungsmittel, Konzentrationen, Reihenfolge der Zugabe u.a.) variiert wurden, führten nicht zum gewünschten Ergebnis.

Auch umfangreiche Versuche, die hochreine Base in für pharmazeutische Zwecke erforderlichen Mengen aus dem entstandenen Produktgemisch durch herkömmliche Verfahren zu reinigen, blieben erfolglos.

Eine Reinigung durch Kristallisation scheidet aus, da die Basen der allgemeinen Formel I, z.B. Fesoterodine, nach dem in EP 1 077 912 beschriebenen Herstellverfahren als viskose Öle vorliegen und aus dem Produktgemisch bisher nicht kristallisiert werden konnten.

Auch Versuche der Reinigung durch Destillation führten nicht zum gewünschten Erfolg.

Eine Reinheit von nur 90-96 Gew % ist aber für pharmazeutische Präparate nicht zufriedenstellend. Vielmehr wird in der Regel eine Reinheit von über 97 Gew.-% bevorzugt. Es bestand daher ein Bedarf an hochreinen freien Basen von 3,3,-Diphenylpropylaminen.

WO 01/35957 lehrt stabile, kristalline Salze von 3-3-Diphenylpropylamin-Derivaten, die gegenüber den amorphen Salzen den Vorteil höherer Stabilität und Reinheit haben.

Derartige Salze sind grundsätzlich zur therapeutischen Verabreichung geeignet und können beispielsweise zur oralen oder parenteralen Therapie eingesetzt werden.

Für einige Anwendungen, z.B. zur transdermalen oder transmukosalen Applikation sind Wirkstoffsalze jedoch in vielen Fällen weniger geeignet, da ihre ionisierte Form die Passage der Haut, bzw. Schleimhaut in therapeutisch effektiver Menge verhindert. Ist eine transdermale oder transmukosale Anwendung gewünscht, muss daher häufig der aminohaltige Wirkstoff in Form der Base appliziert werden.

Überraschenderweise wurde nun gefunden, dass eine freie Base der allgemeinen Formel I (siehe unten) in einer Reinheit von regelmäßig über 97 Gew.-%, bevorzugt über 98 Gew.-%, besonders bevorzugt über 98,5 Gew.-% und ganz besonders bevorzugt über 99 Gew.-% und in guter Ausbeute von über 80 % (Mol-%), in der Regel über 90 % gewonnen werden kann, wenn die freie Base hergestellt wird, in dem sie mit einem geeigneten Reagenz aus einem hochreinen kristallinen Salz freigesetzt wird.

Ein Gegenstand der Erfindung ist daher eine pharmazeutische Formulierung enthaltend eine Verbindung der Formel I 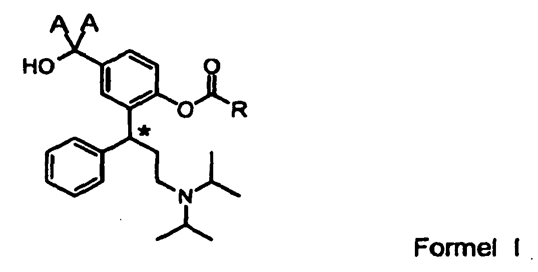 wobei A Wasserstoff oder Deuterium darstellt, R für eine Gruppe steht, die ausgewählt ist aus C₁₋₆Alkyl, C₃₋₁₀ Cycloalkyl oder Phenyl, die jeweils mit C₁₋₃ Alkoxy, Fluor, Chlor, Brom, Iod, Nitro, Amino, Hydroxy, Oxo, Mercapto oder Deuterium substituiert sein kann, wobei das mit "*" (Stern) markierte C-Atom in (R)-Konfiguration, in (S)-Konfiguration oder als Mischung davon vorliegen kann, und einen pharmazeutisch akzeptablen Träger,
dadurch charakterisiert, dass die besagte Verbindung der Formel I als freie Base mit einem Salzgehalt von weniger als 10 Gew.-% und in einem Reinheitsgrad von über 97 Gew.-%, bevorzugt über 98 Gew.%, besonders bevorzugt über 98,5 Gew.-% und ganz besonders bevorzugt über 99 Gew.-% vorliegt.

In einer bevorzugten Ausführungsform ist R ausgewählt aus der Gruppe Methyl, Ethyl, iso-Propyl, 1-Propyl, 1-Butyl, 2-Butyl, tert.-Butyl, iso-Butyl, Pentyl und Hexyl.

In einer besonders bevorzugten Ausführungsform ist R Isopropyl (i-Pr), so daß die in der erfindungsgemäßen pharmazeutischen Formulierung enthaltene Verbindung 2-[3-(1,1-Diisopropylamino)-1-phenylpropyl]-4-(hydroxymethyl)phenyl isobutyrat (Fesoterodine-Base) ist.

In einer Ausführungsform der Erfindung liegen die Verbindungen der allgemeinen Formel 1 in der erfindungsgemäßen Formulierung als Razemate, daß heißt als Mischungen der (R)- und (S)-konfigurierten Moleküle vor.

In einer anderen bevorzugten Ausführungsform liegt das mit "*" (Stern) gekennzeichnete C-Atom in (R)-Form vor, wobei bevorzugt zu über 98 Gew.-% der Verbindung, besonders bevorzugt zu über 99 Gew.-% der Verbindung und ganz besonders bevorzugt zu über 99,5 Gew.-% der Verbindung in (R)-Konfiguration vor.

In einer ganz besonders bevorzugten Ausführungsform enthält die erfindungsgemäße pharmazeutische Formulierung die hochreine freie Base von (R)-2-[3-(1,1-Diisopropylamino)-1-phenylpropyl]-4-(hydroxymethyl)phenyl isobutyrat (Fesoterodine Base), mit einem Reinheitsgehalt von über 97 Gew.-%, bevorzugt über 98 Gew.-%, besonders bevorzugt über 98,5 Gew.-% und ganz besonders bevorzugt über 99 Gew.-%.

In einer bevorzugten Ausführungsform ist der pharmazeutisch akzeptable Träger ein Polymer.

Unter dem Ausdruck "C₁₋₆Alkyl" wird in der vorliegenden Anmeldung eine geradkettige oder verzweigtkettige Kohlenwasserstoffgruppe mit 1-6 C-Atomen verstanden. Bevorzugte C₁₋₆Alkyle sind unsubstituierte gerad- oder verzweigtkettige Gruppen, insbesondere ausgewählt aus der Gruppe Methyl, Ethyl, Propyl, Isopropyl, 1-Butyl, 2-Butyl, Isobutyl, tert.-Butyl, Pentyl und Hexyl.

Unter dem Ausdruck "C3-10 Cycloalkyl" wird eine zyclische Kohlenwasserstoffgruppe mit 3-10 Kohlenstoffatomen verstanden.

Unter dem Ausdruck "hochrein" wird in dieser Anmeldung ein Reinheitsgrad des Monoesters der allgemeinen Formel I von mindestens von über 97 Gew.-%, bevorzugt über 98 Gew.-%, besonders bevorzugt über 98,5 Gew.-% und ganz besonders bevorzugt über 99 Gew.-% verstanden, d.h. es liegt ein entsprechend geringer Anteil an Diester, Dihydroxyverbindungen, 4-Monoestern oder Polymeren vor. Der Reinheitsgrad wird dabei bestimmt wie im Methodenteil beschrieben.

Unter dem Ausdruck freie Base" wird verstanden, dass weniger als 10 Gew%, bevorzugt weniger als 5 % oder 3 %, besonders bevorzugt weniger als 1 % der Verbindung der allgemeinen Formel I in Salzform vorliegt. Der Salzgehalt wird dabei bestimmt wie im Methodenteil beschrieben.

Die in den erfindungsgemäßen pharmazeutischen Formulierungen verwendeten hochreinen Basen der allgemeinen Formel I können hergestellt werden, in dem sie aus hochreinen kristallinen Salzen der allgemeinen Formel 11 freigesetzt werden: 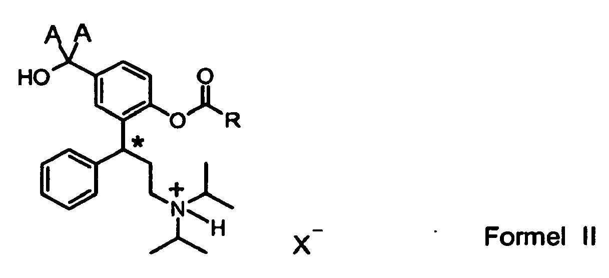 wobei A und R die weiter oben angegebene Bedeutung haben, X- der Säurerest einer physiologisch verträglichen Säure ist, und wobei das mit "***" (Stem) markierte C-Atom in (R)-Konfiguration, in (S)-Konfiguration oder als Mischung davon vorliegen kann.

Als Säurerest X⁻ kommt dabei insbesondere das Anion einer der nachfolgend genannten Säuren in Betracht:

Salzsäure, Bromwasserstoffsäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Äpfelsäure, L-(-)-Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäure (Brenztraubensäure), Furan-2-Carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoyl-glycin), Acetursäure (N-Acetylglycin), Phloretinsäure (3-(4-Hydroxyphenyl)-propionsäure), Phthalsäure, Methansulphonsäure oder Orotsäure, wobei die Säureanionen Hydrogenfumarat und Hydrochlorid besonders bevorzugt werden.

Aus dieser hochreinen Verbindung der allgemeinen Formel 11 werden die korrespondierenden hochreinen freien Basen durch Zugabe geeigneter basischer Reagenzien ("Freisetzungsregenzien") freigesetzt, wobei das
Freisetzungsreagenz ausgewählt ist aus der Gruppe der
- Alkali-, Erdalkali- oder Ammonium-Hydrogencarbonate,
- Amine, Polyamine und basischen Polyaminosäuren und
- basischen Ionentauscher,
einen pK_{B} von 8-11 hat, und
eine Präzipitation der Basen der 3,3-Diphenylpropylaminmonoester im jeweiligen Lösemittel verhindert. Zudem sollte eine Hydrolyse der Esterbindung vermieden werden.

In wässriger Umgebung führt beispielsweise die Umsetzung einer Verbindung der Formel II mit einem Hydrogencarbonat dazu, dass sich zunächst ein wasserlösliches Hydrogencarbonatsalz eines 3,3-Diphenylpropylaminmonoesters als Zwischenprodukt bildet. Beim Ausschütteln mit organischem Lösemittel, z.B. Dichlormethan, entweicht das CO₂ und die schlecht wasserlösliche freie Base des 3,3-Diphenylpropylaminmonoesters lässt sich ohne weitere Reinigung aus der organischen Phase als hochreines Öl gewinnen.

Durch diese Verfahrensführung wird verhindert, dass die Base des 3,3-Diphenylmonoesters unmittelbar nach der Freisetzung präzipitiert, was eine geringere Reinheit und/oder eine geringere Ausbeute zur Folge haben kann. Auch wird eine Hydrolyse der Esterbindung vermieden.

Die Hydrogencarbonatsalze der Verbindungen der allgemeinen Formel I, insbesondere Fesoterodine Hydrogencarbonat, werden als bevorzugte Zwischenprodukte explizit zum Gegenstand der Erfindung gemacht.

Als Freisetzungsreagenz besonders bevorzugt wird daher ein Alkali-, Erdalkali- oder Ammonium-Hydrogencarbonat, wobei Natrium-Hydrogencarbonat ganz besonders bevorzugt wird.

In einem bevorzugten Herstellverfahren der erfindungsgemäßen Formulierung wird daher zunächst ein Salz der Formel II in Wasser aufgenommen und mit einem basischen Freisetzungsreagenz, z.B. einem Hydrogencarbonat, versetzt. Dann wird mit einem geeigneten Lösemittel ausgeschüttelt und die organische Phase eingeengt, bis hochreine Base der Formel I als viskoses Öl zurückbleibt, wobei das Lösemittel ausgewählt ist aus der Gruppe.

Dichlormethan, tert.-Butylmethylether, Diethylether, Ethylacetat, Ethylmethylketon sowie Toluol, wobei Dichlormethan besonders bevorzugt wird.

In einem alternativen Herstellverfahren wird das hochreine Salz der Formel 11 in einem geeigneten Lösemittel aufgenommen und dann über einen Träger geleitet, der beispielsweise immobilisierten Ionentauscher enthält. Das Eluat enthält dann die hochreine Base der allgemeinen Formel I.

Besonders bevorzugt wird zur Herstellung der in der erfindungsgemäßen Formulierung enthaltenen hochreinen freien Base von (R)-2-[3-(1,1-Diisopropylamino)-1-phenylpropyl]-4-(hydroxymethyl)phenyl isobutyrat als Ausgangsverbindung das Hydrogenfumarat von (R)-2-[3-(1,1-Diisopropylammonio)-1-phenylpropyl]-4-(hydroxymethyl)phenyl isobutyrat eingesetzt.

Ein Gegenstand der Erfindung ist daher ein Verfahren zur Herstellung einer pharmazeutischen Formulierung, umfassend die Herstellung einer Verbindung der allgemeinen Formel I 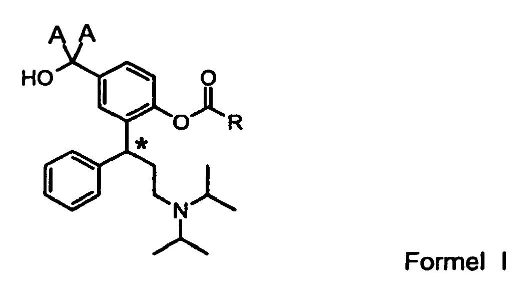 wobei A Wasserstoff oder Deuterium darstellt, R für eine Gruppe steht, die ausgewählt ist aus C₁₋₆ Alkyl, C₃₋₁₀ Cycloalkyl oder Phenyl, die jeweils mit C₁₋₃Alkoxy, Fluor, Chlor, Brom, Iod, Nitro, Amino, Hydroxy, Oxo, Mercapto oder Deuterium substituiert sein kann und wobei das mit "*" (Stern) markierte C-Atom in (R)-Konfiguration, in (S)-Konfiguration oder als Mischung davon vorliegen kann, in einer Reinheit von wenigstens von über 97 Gew.-%, bevorzugt über 98 Gew.-%, besonders bevorzugt über 98,5 Gew.-% und ganz besonders bevorzugt über 99 Gew.-%,
wobei das Verfahren gekennzeichnet ist durch die Freisetzung der hochreinen freien Base der allgemeinen Formel I aus einem kristallinen Salz der allgemeinen Formel II 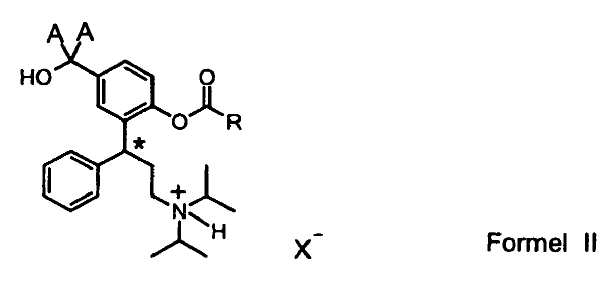 mit einer Reinheit von 97 Gew.-%, bevorzugt über 98 Gew.-%, besonders bevorzugt über 98,5 Gew.-% und ganz besonders bevorzugt über 99 Gew.-%, wobei A und R die oben angegebene Bedeutung haben und X⁻ der Säurerest einer physiologisch verträglichen Säure ist, und wobei das mit "*" (Stern) markierte C-Atom in (R)-Konfiguration, in (S)-Konfiguration oder als Mischung davon vorliegen kann, dadurch gekennzeichnet, dass die Umsetzung der Verbindung der Formel 11 mit einem Freisetzungsreagenz in wässriger Lösung erfolgt, wobei das Freisetzungsreagenz einen pK_{B} von 8 - 11 hat, nicht zur Präzipitation der Verbindungen der Formel 1 führt, und das aus₋gewählt ist aus der Gruppe
(a) der Alkali-, Erdalkali- oder Ammonium-Hydrogencarbonate
(b) der Amine, Polyamine und basischen Polyaminosäuren und
(c) der basischen Ionentauscher,
und das Mischen der so enthaltenen Verbindung mit einem pharmazeutisch akzeptablen Träger.

Bevorzugt wird das erfindungsgemäße Herstellverfahren eingesetzt, um pharmazeutische Formulierungen enthaltend hochreine Basen der allgemeinen Formel I herzustellen, in denen das mit "*" gekennzeichnete C-Atom in (R)-Konfiguration vorliegt und/oder in denen der Substituent R ausgewählt ist aus der Gruppe Methyl, Ethyl, iso-Propyl, 1-Propyl, 1-Butyl, 2-Butyl, tert.-Butyl, iso-Butyl, Pentyl und Hexyl.

Besonders bevorzugt dient das erfindungsgemäße Herstellverfahren zur Herstellung der pharmazeutischen Formulierungen enthaltend eine hochreinen freien Base von (R)-2-[3-(1,1-Diisopropylamino)-1-phenylpropyl]-4-(hydroxymethyl)phenyl isobutyrat, wobei besonders bevorzugt als Ausgangsverbindung der Formel II (R)-2-[3-(1,1-Diisopropylammonio)-1-phenylpropyl]-4-(hydroxymethyl)phenyl isobutyrat hydrogenfumarat eingesetzt wird.

Die Herstellung der hochreinen Salze der Formel 11 ist aus WO 01/35957 bekannt. Hierzu wird zunächst eine Lösung von 2-[3-(1,1-Diiopropylamino)-1-phenylpropyl]-4-(hydroxymethyl)phenol in basischer Lösung mit einem Säurechlorid. z.B. lsobuttersäurechlorid, umgesetzt. Die resultierende Base mit niedrigem Reinheitsgehalt wird dann unter Erwärmen mit einer Säure, z.B. Fumarsäure, versetzt. Das entstandene Salz der allgemeinen Formel II lässt sich in geeigneten Lösungsmitteln auskristallisieren. Die Kristalle werden erneut gelöst und rekristallisiert.

Dieser Prozess kann gegebenenfalls wiederholt werden, bis eine Verbindung der Formel II mit dem gewünschten Reinheitsgrad erhalten wird. Aus diesen Salzen wird die hochreine freie Base der Formel I und die erfindungsgemäße pharmazeutische Formulierung wie oben beschrieben gewonnen.

Die Ausbeute an hochreiner freier Base der Formel I beträgt dabei in der Regel über 90 % der Theorie bezogen auf die Menge des eingesetzten 3,3-Diphenylaminomonoesters der Formel II.

Tabelle 1 zeigt die Aufreinigung von Fesoterodin-Base durch das erfindungsgemäße Verfahren

| **Verfahrensschritte ^{a})** | **Reinheit B oder E (%)** |
|---|---|
| 1. chemische Synthese von B aus A | 94,37 |
| 2. Herstellung des Salzes E aus B (1.) | 92,58 |
| 3. Umkristallsierung des Salzes E aus (2.) | 99,32 |
| 4. Freigesetzte hochreine Base B aus E (3.) | 99,14 |

| | |
|---|---|
| ^{a}) A, B, C, E: R = i-Pr, s. Abbildung 1/4 | |

Die erfindungsgemäßen reinen Basen der allgemeinen Formel I liegen nach der Herstellung in Form eines Öls vor und sind bei -20 °C stabil.

Bei höheren Temperaturen, z.B. bei 2°C-8°C werden die erfindungsgemäßen freien Basen bevorzugt in Gegenwart von Trockungsmitteln gelagert.

Das erfindungsgemäße Verfahren erlaubt erstmals die effiziente Darstellung der pharmazeutischen Formulierung enthaltend eine freien Base der allgemeinen Formel in hochreiner Form. Das Verfahren ist aufskalierbar und ermöglicht die Herstellung hochreiner Verbindungen im gewerblichen Maßstab und stellt erstmals die die hochreinen Basen der allgemeinen Formel I enthaltenden pharmazeutischen Formulierungen zur Verfügung.

Da die in den erfindungsgemäßen pharmazeutischen Formulierungen enthaltenen freien Basen empfindlich für Hydrolysen bzw. für Umesterungen sind, sollte die Lagerung der pharmazeutischen Formulierungen bevorzugt bei < 25°C, besonders bevorzugt bei < 8°C und in Gegenwart von Trocknungsmitteln erfolgen.

Bevorzugt liegen die freien Basen in der erfindungsgemäßen pharmazeutischen Formulierung in leicht saurem Milieu, das heißt bei einem pH von 3-7, bevorzugt pH 3-6 oder pH 3-5 vor, da die Stabilität der freien Basen unter diesen Bedingungen am höchsten ist.

Ferner wird aus Stabilitätsgründen bevorzugt, dass die pharmazeutischen Formulierungen frei von kurzkettigen C₁₋₈ Alkoholen, insbesondere frei von C₁₋₄ Alkoholen sind.

Die Ausgestaltung der pharmazeutischen Formulierung hängt vor allem vom Applikationsweg sowie von den gewünschten Eigenschaften der jeweiligen Verabreichungsform ab.

Möglich sind somit beispielsweise:
➢ Orale Formen: Pulver, Granulate, Tabletten, Dragees, Kapseln, Lösungen oder Suspensionen
➢ parenterale Formen: Lösungen oder Suspensionen
➢ transdermale Formen: Transdermale therapeutische Systeme (TTS), Salben, Cremes, Folien, Lotionen, Sprays, Gele oder Schäume
➢ transmukosale Formen:
   - bukkale oder sublinguale Formen: schnell freisetzende Tabletten, Sprays, Tropfen, Oblaten-förmige Arzneiformen sowie mukoadhäsive Pellets oder Pflaster
   - nasale Formen: Lotionen, Tropfen, Sprays, Salben
   - pulmonale Formen: Aerosole

Als pharmazeutisch akzeptable Trägerstoffe kommen grundsätzlich die einem Fachmann auf dem Gebiet der pharmazeutischen Technologie bekannten Hilfsstoffe in Frage, wie sie beispielsweise in Sucker, Fuchs und Speiser, Pharmazeutische Technologie, Georg Thieme Verlag, Stuttgart, und anderen Übersichtswerken zu entsprechenden Arzneiformen beschrieben sind.

Eine solche pharmazeutische Formulierung kann konventionell sein, kann aber auch in Abhängigkeit von den speziellen Bedürfnissen des Patienten als schnell freisetzende oder retardierte Formulierung ausgestaltet sein.

Die Basen der allgemeinen Formel I, z.B. Fesoterodine, haben sich als überraschend membrangängig herausgestellt. Aus diesem Grund bieten sich zur transdermalen oder transmukosalen Applikation geeignete pharmazeutische Formulierungen besonders an.

Bevorzugt werden die hochreinen Basen der allgemeinen Formel I in den erfindungsgemäßen pharmazeutischen Formulierungen zur transdermalen oder transmukosalen Applikation verwendet, die den Wirkstoff kontrolliert freisetzen.
Besonders bevorzugt werden dabei pharmazeutische Formulierungen, die nach einer anfänglichen Anflutungsphase eine konstante Fluxrate durch Haut oder Schleimhaut eines Patienten über mindestens 24 Stunden, bevorzugt mindestens 48 Stunden gewährleisten.

Zur Gewährleistung solch kontrollierter Wirkstoffabgabe enthält die pharmazeutische Formulierung bevorzugt mindestens eine Polymerschicht, in der eine hochreine Base der allgemeinen Formel I dispergiert oder gelöst ist.

Über die Zusammensetzung einer solchen Polymerschicht kann Einfluß auf das Freisetzungsverhalten des Wirkstoffs genommen werden. So bestimmt beispielsweise das Löslichkeitsverhalten von Wirkstoffen in der Polymermatrix maßgeblich die Freisetzung des Wirkstoffs aus transdermalen/transmukosaten therapeutischen Systemen und somit auch die Fluxraten durch Haut oder Schleimhaut.

Ferner kann die Polymerschicht haftklebende Substanzen enthalten, die die Fixierung der pharmazeutischen Zusammensetzung auf der Haut oder der Schleimhaut des Patienten ermöglicht.

Beispielsweise kann eine bukkale Formulierung als mukoadhäsives System ausgestaltet sein, aus dem der Wirkstoff protahiert freigesetzt wird. Zur Adhäsion an die Mukosa werden adhesive Polymere/Copolymere verwendet, wie beispielsweise PVP, Pektine, Carbopol, Polyacrylate, Cellulose-Derivate, Chitosan oder Polyoxyethylen. Entsprechende Beispiele und Übersichten finden sich zum Beispiel in US 6210699; US 4855142; US 4680323; US 5700478; US 4948580; US 4715369; US 4876092; US 5750136; Woodley, Clin Pharmacokinet 40, 2001, 77 oder Singla, Drug Dev Ind Pharm 26 (2000) 913. Diese adhäsiven Polymere/Copolymere können als adhesive Hüllschicht, z.B. von Tabletten, fungieren, können jedoch, z.B. in einem Bukkal-Pflaster, auch Bestandteil einer adhesiven Polymermatrix sein, in der der Wirkstoff gelöst oder dispergiert vorliegt (Wong, Int J Pharm. 178, 1999, 11).

In einer Ausführungsform der Erfindung ist die pharmazeutische Formulierung zur transdermalen Verabreichung einer hochreinen Base der Formel I daher als bukkale Formulierung, insbesondere als Bukkal-Pflaster ausgebildet, das mindestens eine Polymerschicht umfasst, in der die hochreine Base der allgemeinen Formel I gelöst oder dispergiert vorliegt. Diese die hochreine Base enthaltende Polymerschicht hat bevorzugt mukoadhäsive Eigenschaften.

In einer besonders bevorzugten Ausführungsform der Erfindung ist die pharmazeutische Formulierung zur transdermalen Verabreichung einer hochreinen Base der Formel I als transdermales Pflaster ausgebildet.

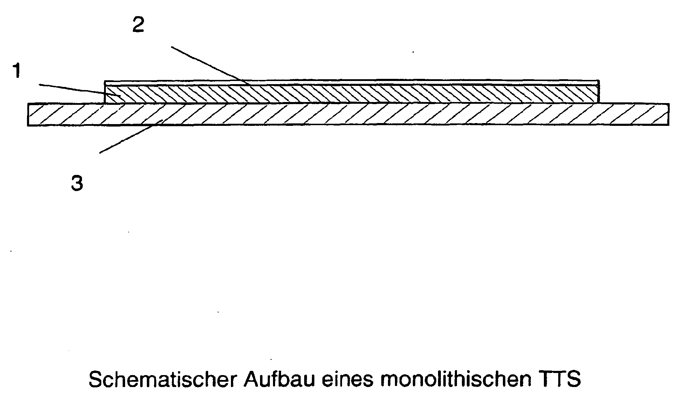

Transdermale Pflaster (oft auch als transdermale therapeutische Systeme, TTS, bezeichnet) können in verschiedener Weise kategorisiert werden, wobei häufig die folgenden drei Hauptgruppen unterschieden werden:
- Der Reservoir-Typ, in dem der Wirkstoff in Lösung oder in einem Gel vorliegt und durch eine Geschwindigkeits-regulierende Membran an die Haut des Patienten gebracht wird.
- Der Matrix-Typ, der weiter unterteilt werden kann in
   ➢ den Laminat-Typ, in dem der Wirkstoff in einer Schicht (Matrix) nicht-adhesiven Polymers vorliegt. Das TTS kann weitere Schichten zur Befestigung an der Haut, z.B. eine Kleberschicht, enthalten, kann jedoch auch durch separate Klebstofffolien ("overtapes") an der Haut fixiert werden.
   ➢ den monolithischen Typ, in dem der Wirkstoff in einer Haftkleberschicht (Klebermatrix) vorliegt. Ein Beispiel für einen typischen Aufbau eines monolithischen TTS ist in Abbildung 4/4 wiedergegeben. Das beispielhafte monolithische TTS besteht aus der wirkstoffhaltigen Klebermatrix (1), einer für die Inhaltsstoffe der Klebermatrix inerten und undurchlässigen Rückschicht, die sich nach dem Auftrag des TTS auf der Haut abgewandten Seite des TTS befindet (2) sowie einer ablösbaren Schicht, die vor dem Auftrag des TTS auf die Haut entfernt wird (3).
- Iontophoretische Systeme, in denen der Wirkstofftlux durch die Haut durch das Anlegen elektrischer Spannung unterstützt wird.

Besonders bevorzugte Arzneimittel im Sinne der vorliegenden Erfindung sind TTS vom Matrix-Typ, wobei monolithische TTS, in denen der Wirkstoff in der Klebermatrix vorliegt, ganz besonders bevorzugt werden.

In der vorliegenden Patentanmeldung wird nachfolgend unter dem Begriff "Polymermatrix" oder "Matrix" eine polymerhaltige Schicht oder Masse verstanden, wobei der Begriff "Klebermatrix" davon umfasst ist.

Unter dem Ausdruck "Gesamtgewicht der Polymermatrix" wird in dieser Patentanmeldung das Gewicht der Polymermatrix inklusiv des darin eingebrachten Wirkstoffs und etwaiger Hilfsstoffe verstanden.

Nicht-limitierende Beispiele für adhesive Polymere/Copolymere, die zur Herstellung transdermaler Vorrichtungen geeignet sind und die den Wirkstoff der allgemeinen Formel I in gelöster, teilweise gelöster oder dispergierter Form enthalten können, sind Silikonkleber, Ethlvinylacetat (EVA-)-Kleber, Styrol-Block-Copolymer (SXS)-Kleber, Acrylatkleber, Polyurethankleber, Vinylacetatkleber sowie adhesive Gummen, z.B. Polyisobutylene, Polybutadiene, Neoprene oder Polyisoprene sowie geeignete Mischungen dieser Kleber.

Als Haftkleber besonders geeignet sind die in der Pflastertechnologie bekannten Polymer-Kleber vom Silikon-Typ, vom Acrylat-Typ, vom SxS-Typ sowie vom Ethylvinylacetat-Typ (EVA-Typ), die aus dem Stand der Technik bekannt sind. Die Eigenschaften dieser Haftkleber werden weiter unten näher beschrieben.

Die Dosierung der erfindungsgemäßen Verbindungen ist abhängig von Alter, Gewicht und Zustand des Patienten, Applikationsart und -intervall. Im allgemeinen liegt die effektive Tagesdosis im Bereich von 0,5-20 mg. Typischerweise werden bei oraler Gabe mindestens 3 mg/Tag, z.B. 3-15 mg/Tag, bevorzugt 4-12 mg/Tag eingesetzt. Eine typische transdermale oder transmukosale Tagesdosis, z.B. für Fesoterodine, für einen erwachsenen Patienten liegt beispielsweise bei mindestens 3 mg, bevorzugt im Bereich 3-15 mg und besonders bevorzugt zwischen 4 und 12 mg.

Eine pharmazeutische Zusammensetzung, die zur einmal täglichen Gabe geeignet ist, sollte daher bevorzugt 3-15 mg einer hochreinen Base der allgemeinen Formel1 enthalten.

Ist die pharmazeutische Zusammensetzung eine transdermale Formulierung, enthält diese aus Sicherheitsgründen im allgemeinen etwa das zweifache der zu verabreichenden Wirkstoffmenge. Eine typische erfindungsgemäße Formulierung zur transdermalen Verabreichung einer hochreinen Verbindung der allgemeinen Formel 1 enthält somit mindestens 6 mg Wirkstoff, kann je nach Dosierungshöhe und Applikationsintervall aber auch mehr als 10 mg, 20 mg, 30 mg, 40 mg oder 50 mg hochreinen Wirkstoff der allgemeinen Formel 1, z.B. Fesoterodine pro Dosierungseinheit enthalten. Ist ein ein fünf- oder sogar 7 tägiges Applikationsintervall geplant, kann der Wirkstoffgehalt einer Einzeldosierungseinheit auch bei über 70, 80, 90 oder sogar über 100 mg liegen.

Unter "Dosierungseinheit" wird in dieser Patentanmeldung eine pharmazeutische Formulierung verstanden, die eine definierte Menge Wirkstoff enthält und diesen nach einmaliger Administration beim Patienten über einen vorbestimmten Zeitraum in therapeutisch effektiver Menge freisetzt. Der Begriff "Dosierungseinheit" umfasst somit in dieser Patentanmeldung sowohl eine Tablette zur dreimal täglichen Applikation als auch ein Pflaster zur wöchentlichen Verabreichung.

Ein Gegenstand der vorliegenden Erfindung ist somit eine pharmazeutische Formulierung ausgebildet als Dosierungseinheit, die mindestens 3 mg einer Verbindung der allgemeinen Formel I, 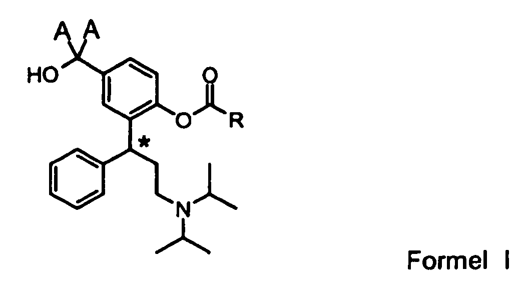 sowie mindestens einen pharmazeutisch akzeptablen Trägerstoff enthält, wobei A Wasserstoff oder Deuterium ist, R für eine Gruppe steht, die ausgewählt ist aus C₁₋₆ Alkyl, C₃₋₆ Cycloalkyl oder Phenyl, die jeweils mit C₁₋₃ Alkoxy, Fluor, Chlor, Brom, Iod, Nitro, Amino, Hydroxy, Oxo, Mercapto oder Deuterium substituiert sein kann und wobei das mit "*" (Stern) markierte C-Atom in (R)-Konfiguration, in (S)-Konfiguration oder als Mischung davon vorliegen kann, und wobei die freie Base der Verbindung I mit einem Salzgehalt von weniger als 10 Gew.-% und in einer Reinheit von über 97 Gew.-%, bevorzugt über 98 Gew.-%, besonders bevorzugt über 98,5 Gew.-% und ganz besonders bevorzugt über 99 Gew.-% vorliegt.

In anderen Ausführungsformen der Erfindung enthält die Dosierungseinheit mindestens 10 mg, 20 mg, 30 mg, 40 mg, 50 mg, 70 mg, 80 mg, 90 mg oder sogar über 100 mg hochreinen Wirkstoff der allgemeinen Formel 1.

In einer bevorzugten Ausführungsform der Erfindung enthält die erfindungsgemäße Dosierungseinheit eine Verbindung der allgemeinen Formel I, in der R ausgewählt ist aus der Gruppe Methyl, Ethyl, iso-Propyl (i-Pr), 1-Propyl, 1-Butyl, 2-Butyl, tert.-Butyl, iso-Butyl, Pentyl und Hexyl, wobei R besonders bevorzugt Isopropyl ist und wobei das mit "*" (Stern) gekennzeichnete C-Atom besonders bevorzugt in (R)-Konfiguration vorliegt.

In einer ganz besonders bevorzugten Ausführungsform der Erfindung enthält die Dosierungseinheit die freie Base von (R)- 2-[3-(1,1-Diiopropylamino)-1-phenylpropyl]-4-(hydroxymethyl)phenyl isobutyrat (Fesoterodin freie Base) in einem Reinheitsgrad von mindestens 97 Gew.-%, bevorzugt über 98 Gew.-%, besonders bevorzugt über 98,5 Gew.-% und ganz besonders bevorzugt über 99 Gew.-%

Ist die pharmazeutische Formulierung als transdermale Verabreichungsform ausgebildet, sollte die Fluxrate durch die Haut des Patienten möglichst konstant sein, um Konzentrationsschwankungen im Plasma zu vermeiden.

Die Tagesdosis sollte daher bei einer Auftragsfläche von 50 cm², bevorzugt maximal 40 cm² in einem steady state Flux durch Humanhaut von mehr als 6 µg/cm²/h, bevorzugt von mehr als 8 µg/cm²/h, besonders bevorzugt von mehr als 10 µglcm²/h und ganz besonders bevorzugt von mehr als 12 µg/cm²/h verabreicht werden, wobei die Fluxraten in einem in-vitro Humanhautmodell nach Tanojo bestimmt werden, wie in Ausführungsbeispiel 3.2 beschrieben.

Die Erfindung betrifft auch die Herstellung von Arzneimitteln.

Die in den erfindungsgemäßen pharmazeutischen Formulierungen enthaltenden hochreinen Basen sind zur Verwendung bei der Herstellung eines Arzneimittels, besonders zur Behandlung von Inkontinenz, ganz besonders zur Behandlung von DrangInkontinenz sowie zur Behandlung der Hyperaktivität des Detrusors, der Hyperaktivität der Blase, von Pollakisurie, Nykturie oder imperativem Harndrang geeignet.

Die die hochreinen Basen der Formel I enthaltenden pharmazeutischen Formulierungen können beispielsweise zur Herstellung bukkal verfügbarer Arzneimittel, z.B. Sprays, mucoadhäsiver Pellets oder schnell auflösender Oblaten, wie z.B. in WO 02/02085 beschrieben, verwendet werden.

Andere bevorzugte Arzneiformen der transdermale Formulierungen, z.B. Salben, Cremes, Lotionen, Sprays, Pasten, Folien oder wirkstoffhaltige Pflaster.

Dabei wird die hochreine Base der allgemeinen Formel 1 bevorzugt zur Herstellung eines Arzneimittels zur retardierten transdermalen oder transmukosalen Verabreichung verwendet und zu diesem Zweck bevorzugt in eine adhesive oder nicht-adhesive Polymermatrix eingebracht.

Die hochreine Verbindung der allgemeinen Formel I liegt in der erfindungsgemäßen pharmazeutischen Formulierung in Form der freien Base mit einem gebundenen Salzanteil von weniger als 10 Gew%, besonders bevorzugt weniger als 5 % oder 3 %, ganz besonders bevorzugt weniger als 1 % vor.

Die aus WO 01/35957 bekannten hochreinen Salze von 3-3-Diphenylpropylamin-Derivaten, z.B. das Fumaratsalz von Fesoterodine führen bei transdermaler Verabreichung nur zu therapeutisch unzureichenden Fluxraten. Auch der Zusatz geladener Moleküle, wie z.B. Silicate oder Chitosan, oder von Hautpenetrationsverstärkern, wie Ölsäure oder PGML zu den Wirkstoffsalz-haltigen Matrices führt nicht zu zufriedenstellenden Fluxraten (Tabelle 2).

Auch eine in-situ Freisetzung der Base aus dem korrespondierenden Salz durch die Zugabe von Calciumsilikat während der Herstellung der Klebermatrix, wie in WO 94/07486 beschrieben, führt nicht zu den gewünschten Fluxraten durch Humanhaut (Tabelle 2), da die in-situ Umsetzung zur freien Base im allgemeinen nicht vollständig verläuft, so daß ein zu großer Anteil des Wirkstoffs in protonierter Form in der Matrix vorliegt.

Bei der Herstellung der erfindungsgemäßen Vorrichtungen sollte die Verbindung der allgemeinen Formel I daher der Polymermatrixmasse bereits in Form der hochreinen freien Base zugesetzt werden.

**Tabelle 2:**

| Lot-No | Haftkleber | Verfahren | Wirkstoffbeladung (Gew% Fesoterodin) | Matrixgewicht (g/m²) | Flux µg/cm²/Tag (im steady state; nach 24 h) | |
|---|---|---|---|---|---|---|
| | | | | | Mäusehaut | Humanhaut |
| 20111080¹ | Acrylat | Lösungsmittel | 15 | 100 | 705 | n.b. |
| 20302060¹ | Acrylat | Lösungsmittel | 15 | 87 | n.b. | 332,64 |
| 20111085¹ | EVA | Heißschmelz | 15 | 84 | 510 | 323,7 |
| 20111086¹ | Silikon | Heißschmelz | 15 | 63 | 495 | n.b. |
| 20302062¹ | Silikon | Heißschmelz | 15 | 100 | n.b. | 544,89 |
| 20111087¹ | SxS | Heißschmelz | 15 | 89 | 460 | 383,8 |
| 20302063¹ | Silikon + PVAc⁶ | Heißschmelz | 15 | 83 | n.b. | 501,09 |
| 20002031² | Acrylat | Lösungsmittel | 15 Fumarat | 105 | 27 | n.b. |
| 20104035^{2,3} | Acrylat/OL | Lösungsmittel | 15 Fumarat | 110 | 84 | n.b. |
| 20106061⁴ | Silicon | Lösungsmittel | 15 Fumarat | 60 | n.b. | 24,2 |
| 20106043⁵ | Silikon | Heißschmelz | 15 DiOH⁵ | 101 | n.b. | 2,3 |

| | | | | | | |
|---|---|---|---|---|---|---|
| n.b.=nicht bestimmt; ¹=Zur Matrix wurde Fesoterodin als freie Base zugesetzt; ²=Vergleichsbeispiel hergestellt durch Verwendung des Fesoterodin-Fumaratsalzes; ³=Vergleichsbeispiel hergestellt durch Verwendung des Fesoterodin-Fumaratsalzes mit Ölsäure als Permeationsenhancer; ⁴=Vergleichsbeispiel hergestellt durch in-situ Freisetzung der Base aus dem Fumaratsalz in der Klebermatrix; ⁵=Vergleichsbeispiel hergestellt durch Verwendung des Dihydroxymetaboliten (2-[3-(1,1-Diisopropylamino)-1-phenylpropyl]-4-(hydroxymethyl)phenol) von Fesoterodin; ⁶PVAc = Polyvinylacetat. | | | | | | |

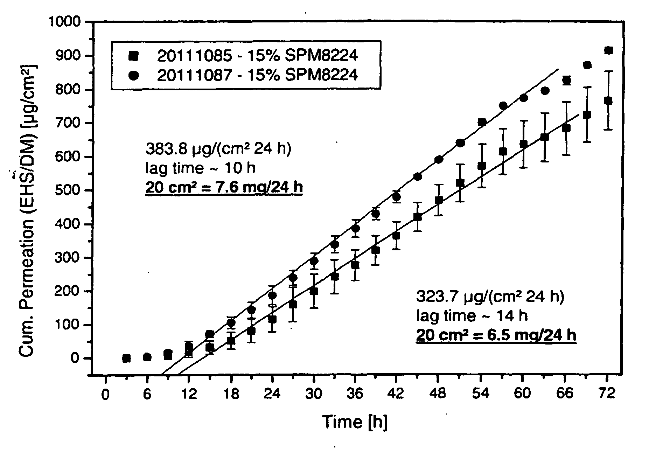

Abbildung 2 zeigt, dass TTS, bei denen die hochreine Base von (R)- Fesoterodine in einer Menge von 15 Gew.-% in geeignete Klebermatrices vom SXS- oder EVA-Typ eingebracht wurde, bei in-vitro Versuchen mit Humanhaut zu Fluxraten führen, die bei entsprechender Auftragsfläche von 5-50 cm² therapeutisch gewünschte Tagesdosen wie folgt ermöglichen (Tabelle 3):

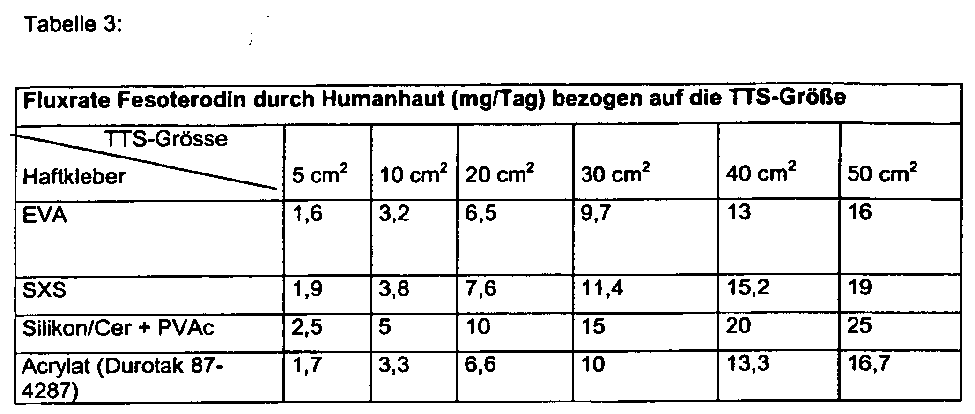

Das verwendete in-vitro Modell nach Tanojo (J. Control Rel. 45 (1997) 41-47) hat sich als ausgezeichnetes Modell erwiesen, in der die gemessenen in-vitro Fluxraten hervorragend mit den in-vivo Fluxraten korrelierten, die in verschiedenen klinischen Studien gemessen wurden. Daraus ergibt sich, dass die therapeutisch gewünschten täglichen Wirkstoff-Fluxraten von mindestens 3 mg, z.B. 3-15 mg, bevorzugt von 4-12 mg oder 6-12 mg durch die Verwendung der erfindungsgemäßen TTS erreicht werden kann.

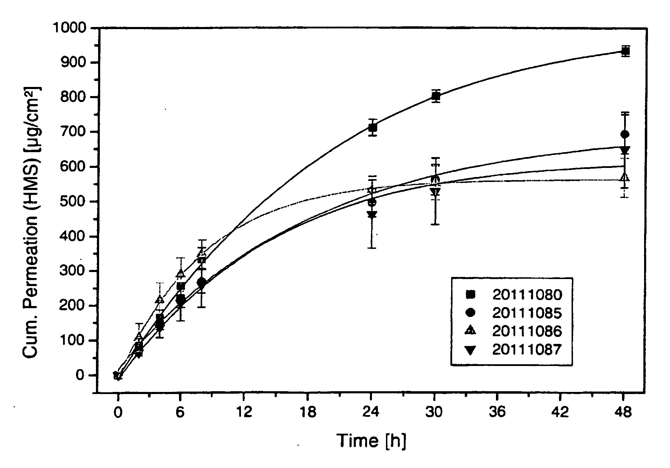

Auch aus Acrylat und Silikon-basierten Matrices konnten in-vitro vergleichbare Fluxraten von Fesoterodin (hochreine freie Base) durch Säugerhaut erreicht werden (Abbildung 3, Tabelle 2).

In besonders bevorzugten Ausführungsformen enthält das Arzneimittel als aktiven Wirkstoff die hochreine Base von Fesoterodin.

In einer besonders bevorzugten Ausführungsform betrifft die Erfindung daher die Verwendung der pharmazeutischen Formulierungen, enthaltend eine hochreine Verbindung der allgemeinen Formel I zur Herstellung eines Arzneimittels, wobei das Arzneimittel
(a) eine selbstklebende Polymerschicht umfasst, die die hochreine Base von Fesoterodin enthält und
(b) die hochreine Base von Fesoterodin mit einer Fluxrate von 0,5 -20 mg/Tag, bevorzugt von mindestens 3 mg/Tag, z.B. 3-15 mg/Tag, besonders bevorzugt 4-12 mg/Tag durch Humanhaut abgibt.

Wie Abbildung 2 zeigt, erfolgt die Wirkstoffabgabe aus solchen Arzneimitteln nach einer ersten Anflutungsphase über wenigstens 24 Stunden weitgehend konstant.

In einer anderen besonderen Ausführungsform betrifft die Erfindung daher die Verwendung einer pharmazeutischen Formulierung, enthaltend eine hochreine Verbindung der allgemeinen Formel I, z.B. Fesoterodin zur Herstellung eines Arzneimittels, wobei das Arzneimittel nach initialer Anflutungsphase die Lösung der allgemeinen Formel I über mindestens 24 Stunden, bevorzugt 36 oder 48 h in konstanter Fluxrate freisetzt.

Unter dem Ausdruck "steady-state" wird in der vorliegenden Patentanmeldung ein Fließgleichgewicht verstanden, das sich nach einer initialen lag-Phase nach dem erstmaligen Auftragen der erfindungsgemäßen Vorrichtung einstellt.

Unter "steady-state Fluxrate" wird eine Fluxrate verstanden, die sich nach der initialen lag Phase einstellt.

Unter dem Ausdruck "konstante Fluxrate" wird in dieser Patentanmeldung eine steady-state Fluxrate verstanden, bei der eine Verbindung der allgemeinen Formel 1 in einer mittleren Fluxrate durch Humanhaut transportiert wird, die eine intraindividuelle Variabilität CV über die Zeit von maximal 30 %, bevorzugt maximal 20 % aufweist, wobei CV nach der Gleichung CV = (sd : x̅) x 100 % bestimmt wird (siehe Berechnung Cawello (ED) in "Parameters for Compartment-free Pharmacokinetics", Shaker Verlag, Aachen, 1999, Seite 112). Eine Tagesdosis wird dabei in einer mittleren Fluxrate von Tagesdosis:24 (mg/Stunde) mit einer CV von 30 % verabreicht. Für den Fachmann ist klar, dass sich eine konstante Fluxrate erst nach einer initialen Anflutungsphase ("lag-Phase") nach erstmaligem Auftrag der Vorrichtung einstellt. Die lag-Phase wird daher bei der Berechnung der konstanten Fluxrate nicht berücksichtigt.

Unter dem Ausdruck "Fluxrate durch Humanhaut" wird in dieser Patentanmeldung, sofern nicht ausdrücklich anders angegeben, eine Fluxrate verstanden, die im in vitro-Humanhautmodell nach Tanojo, wie in Ausführungsbeispiel 3.2 beschrieben, gemessen wurde.

Bevorzugte Polymermatrices sind selbstklebende Polymermatrices vom EVA-, SXS-, Silikon oder Acrylattyp, deren Eigenschaften und Herstellung im folgenden näher beschrieben werden:

### Silikonkleber:

Bevorzugte Silikonhaftkleber sind amin-resistente, druck-sensitive Polyorganosiloxankleber.

Silikonhaftkleber stellen in den meisten Fällen Polydimethylsiloxane dar, allerdings können prinzipiell statt Methylgruppen auch andere organische Reste, wie z.B. Ethyl oder Phenylgruppen vorhanden sein. Aminresistente Silikonhaftkleber zeichnen sich im allgemeinen dadurch aus, dass sie keine oder nur wenige freie Silanolfunktionen enthalten, da die Si-OH-Gruppen alkyliert wurden. Solche Kleber sind in der EP 180 377 beschrieben. Besonders bevorzugte Kleber sind Kondensate oder Mischungen von Silikonharzen und Polyorganosiloxanen, wie beispielsweise in US RE 35 474 beschrieben.

Geeignete Kleber werden beispielsweise von Dow Corning als sogenannte Bio-PSA Kleber vertrieben. Besonders geeignet sind dabei Mischungen der Haftkleber Bio PSA Q7-4301 und Q7-4201 insbesondere im Verhältnis 40:60 bis 60:40.

Pflaster-Matrices auf Basis von Silikonkleber werden überwiegend in lösungsmittelbasierten Verfahren verarbeitet. Hierzu wird im ersten Schritt eine Lösung aus Haftklebern und Wirkstoff in einem organischen Lösungsmittel oder einem Lösungsmittelgemisch hergestellt. Im zweiten Schritt wird die Lösung ausgestrichen und laminiert und dann das Lösungsmittel entfernt. Ein solches Verfahren ist beispielsweise beschrieben in WO 99/49852.

Ein alternatives Verfahren, dass auf die Verwendung von organischen Lösungsmitteln verzichtet, ist das Heißschmelzverfahren. Bei diesem Verfahren wird das Polymer, bzw. der Haftkleber bei Temperaturen zwischen 70 und 200°C, bevorzugt zwischen 90 und 160°C und besonders bevorzugt zwischen 100 und 150 °C geschmolzen und der Wirkstoff in die homogenisierte Matrixschmelze gebracht. Nach kurzer Homogenisierung wird die wirkstoffhaltige Klebermatrix wieder abgekühlt, so daß der Wirkstoff im allgemeinen für weniger als 5 Minuten, falls gewünscht sogar weniger als 4, 3, 2 oder sogar weniger als 1 Minute thermischer Belastung ausgesetzt wird. Danach liegt der Wirkstoff in der erstarrten Polymerschmelze vor. Während des Prozesses ist der Wirkstoff von kritischen Umwelteinflüssen (Licht, Sauerstoff) weitgehend abgeschirmt.

Dieses Verfahren hat gegenüber dem lösungsmittel-basierten Verfahren den Vorteil, dass die hochreinen Basen der allgemeinen Formel I keinen Lösemitteleinflüssen ausgesetzt werden, sondern sofort in die heiße Schmelze gegeben werden können, wo sie nach kurzer Homogenisierung in der erkaltenden Polymermatrix stabilisiert werden. Das Heißschmelzverfahren wird bevorzugt in einem Extruder, z.B. in einem Doppelschneckenextruder durchgeführt, wie in WO 99/48493 beschrieben.

Silikonkleber sind bei den oben genannten Verarbeitungstemperaturen im allgemeinen zu viskos, d.h. haben eine dynamische Viskosität von über 150 Pa.s. In der Patentliteratur wurden verschiedene Verfahren beschrieben, die Viskosität von Silikonklebern durch Beimischung von geeigneten Zusätzen (Weichmachern) heißschmelzfähig zu machen. Beispiele für solche Weichmacher für Silikone sind Glycerolmonolaurat oder Laurylactetat, wie in EP 835 136 beschrieben, Wachse der Formel R-C(O)-OR' wie in EP 360 467 beschrieben, Alkylmethylsiloxanwachse wie in EP 524 775 beschrieben, siloxierte Polyetherwachse, wie in EP 663 431 beschrieben oder organische Wachse, wie in US RE 36 754 beschrieben.

Die Weichmacher werden dem Silikonkleber im allgemeinen in einer Menge von 1-30 Gew.-% bezogen auf das Gesamtgemisch der heißschmelzfähigen Klebermischung zugesetzt. Bevorzugte Weichmacher sind organische Wachse, wie in US RE 36 754 beschrieben, z.B. Ozokerit, Ceresin, Paraffin, Candelila, Carnauba, Bienenwachs oder Mischungen dieser Wachse, wobei Ozokerit und Ceresin ganz besonders bevorzugt werden.

Vorgefertigte heißschmelzfähige Silikonhaftkleber, speziell Mischungen aus Silikonhaftklebern mit Ceresin oder Ozokerit können bei Dow Coming, Michigan, bezogen werden. Durch den Zusatz von 10 Gew.-% Ceresin zu einem Silikon-Haftkleber gelang es beispielsweise, die dynamische Viskosität des resultierenden Haftklebergemischs bei einer Verarbeitungstemperatur von 150°C von über 150 Pa.s auf unter 50 Pa.s zu senken. Eine solche silikonbasierte Haftklebermischung kann in einem Temperaturbereich von 70°C bis 200°C und insbesondere im Bereich zwischen 100°C und 150°C gut im Heißschmelzverfahren verarbeitet werden.

Überraschenderweise wurde festgestellt, daß heißschmelzfähige Silikonhaftkleber ausgezeichnet zur transdermalen Verabreichung der Verbindungen der allgemeinen Formel I geeignet sind.

Ein Gegenstand der Erfindung ist daher eine Vorrichtung zur transdermalen Verabreichung einer Verbindung der Formel I 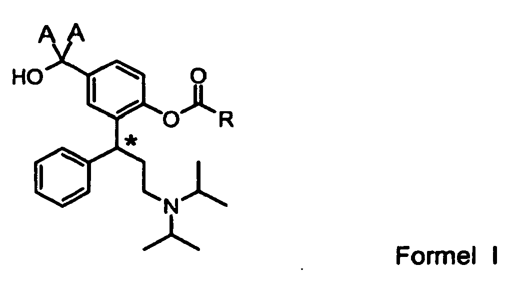 wobei A Wasserstoff oder Deuterium darstellt, R für eine Gruppe steht, die ausgewählt ist aus C₁₋₆Alkyl, C₃₋₁₀ Cycloalkyl oder Phenyl, die jeweils mit C₁₋₃ Alkoxy, Fluor, Chlor, Brom, Iod, Nitro, Amino, Hydroxy, Oxo, Mercapto oder Deuterium substituiert sein kann, wobei das mit "*" (Stern) markierte C-Atom in (R)-Konfiguration, in (S)-Konfiguration oder als Mischung davon vorliegen kann,
dadurch charakterisiert, dass die Verbindung der allgemeinen Formel I in Form der freien Base mit einem Salzgehalt von weniger als 10 Gew.-% in einer Reinheit von über 97 Gew.-%, bevorzugt über 98 Gew.-%, besonders bevorzugt über 98,5 Gew.-% und ganz besonders bevorzugt über 99 Gew.-% in eine selbstklebende Polymerschicht (Klebermatrix) eingebracht wurde, wobei die Klebermatrix ein aminoresistentes Silikon umfasst.

In einer besonders bevorzugten Ausführungsform der Erfindung basiert die Klebermatrix auf einer heißschmelzfähigen Mischung aus einem silikonbasierten Haftkleber und mindestens einem Weichmacher, z.B. Ozokerit. Bevorzugt wurde in die erfindungsgemäße silikon-basierte Matrix als Wirkstoff die hochreine freie Base von Fesoterodin eingebracht.

Unter "heißschmelzfähig" wird dabei verstanden, dass der Haftkleber bei einer im Heißschmelzverfahren üblichen Arbeitstemperatur von z.B. 160°C eine dynamische Viskosität von höchstens 150, bevorzugt höchstens 120 Pa.s aufweist.

Ein weiterer Aspekt der Erfindung ist ein Arzneimittel zur transdermalen Verabreichung einer Verbindung der allgemeinen Formel 1, umfassend eine Klebermatrix, die umfasst:
(a) 50-99 Gew.-% einer Haftklebermischung bestehend aus
   (i) 70-99 Gew.-% eines amin-resistenten Silikonklebers
   (ii) 1-30 Gew.-%, bevorzugt 3-15 Gew.-% eines geeigneten Weichmachers, bevorzugt eines organischen Wachs, welches besonders bevorzugt ausgewählt wird aus der Gruppe Ozokerit, Ceresin, Paraffin, Candelilla, Carnauba, Bienenwachs oder Mischungen dieser Wachse, wobei Ozokerit und Ceresin besonders bevorzugt werden
(b) 1-40 Gew.-% einer Verbindung der allgemeinen Formel 1, die in Form der hochreinen freien Base in die Matrix eingebracht wird.

Silikon-Haftkleber sind kommerziell erhältlich und werden beispielsweise von Dow Corning als Bio-PSA Q7-4300 oder Bio-PSA Q7-4200 vertrieben. Heißschmelzfähige Silikonhaftkleber, bestehend aus Mischungen von PSA 7-4300 mit organischen Wachsen wie Ozokerit oder Ceresin, sind ebenfalls bei Dow Corning erhältlich.

Abbildung 3/4 zeigt den in-vitro Flux durch Mäusehaut, der mit einem im Heißschmelzverfahren hergestellten Silikon-basierten Pflaster, das Ozokerit als Weichmacher für die Klebermatrix enthält und das die hochreine freie Base von Fesoterodin in der Klebematrix enthält, erreicht wurde.

### EVA-Kleber

EVA-Haftkleber sind heißschmelzfähige Haftkleber, die auf Ethylenvinyalacetat-Coplymeren beruhen ("EV A-Haftkleber"). Solche EVA-Kleber werden beispielsweise beschrieben in US 4,144,317. EVA-Kleber zeichnen sich aus durch gute Klebeeigenschaften, einfache Herstellung und Verarbeitung sowie gute Hautverträglichkeit. EVA-Kleber können bezogen werden, z.B. bei Beardow Adams (13/BA).

Zur Verarbeitung im Heißschmelzverfahren gilt grundsätzlich das unter Silikonen gesagte, wobei den EVA-Haftklebern im allgemeinen keine Weichmacher zugesetzt werden müssen.

Ein Gegenstand der Erfindung ist daher eine Vorrichtung zur transdermalen Verabreichung einer Verbindung der Formel I 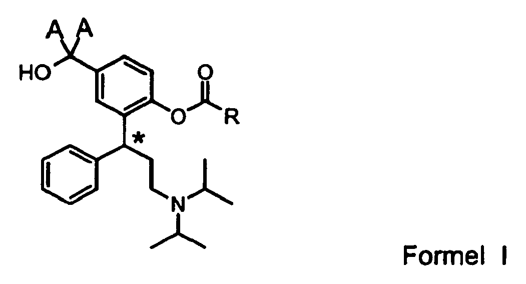 wobei A Wasserstoff oder Deuterium darstellt, R für eine Gruppe steht, die ausgewählt ist aus C1-6 Alkyl, C3-10 Cycloalkyl oder Phenyl, die jeweils mit C1-3 Alkoxy, Fluor, Chlor, Brom, Iod, Nitro, Amino, Hydroxy, Oxo, Mercapto oder Deuterium substituiert sein kann, wobei das mit "*" (Stern) markierte C-Atom in (R)-Konfiguration, in (S)-Konfiguration oder als Mischung davon vorliegen kann,
dadurch charakterisiert, dass die Verbindung der allgemeinen Formel 1 in Form der freien Base mit einem Salzgehalt von weniger als 10 Gew.-% in einer Reinheit von über 97 Gew.-%, bevorzugt über 98 Gew.-%, besonders bevorzugt über 98,5 Gew.-% und ganz besonders bevorzugt über 99 Gew.-% in eine selbstklebende Polymerschicht (Klebermatrix) eingebracht wurde, wobei die Klebermatrix einen Haftkleber vom EVA-Typ enthält.

In einer besonders bevorzugten Ausführungsform der Erfindung ist die EVA-basierte Klebermatrix im Heißschmelzverfahren hergestellt worden. Bevorzugt wurde in die erfindungsgemäße EVA-basierte Matrix als Wirkstoff die hochreine freie Base von Fesoterodin eingebracht.

Abbildungen 2 und 3 zeigen die in-vitro Fluxraten durch Humanhaut, bzw. Mäusehaut, die mit einem im Heißschmelzverfahren hergestellten EVA-basierten Pflaster, das die hochreine freie Base von Fesoterodin in der Klebematrix enthält, erreicht wurden.

### SxS-Haftkleber

SxS Haftkleber können im lösungsmittel-basierten Herstellverfahren wie im Heißschmelzverfahren verarbeitet werden. Unter dem Begriff "SxS Haftkleber" werden in der vorliegenden Patentanmeldung Styrol-Block-Copolymer-basierte Kleber verstanden, die nicht-elastomere Styrolblöcken an den Enden und elastomere Blöcke in der Mitte tragen. Die elastomeren Blöcke können beispielsweise aus Polyethylenbutylen, Polyethylenpropylen, Polybutadien, Polyisobutylen oder Polyisopropen bestehen.

Geeignete SxS-Kleber werden beispielsweise in US 5,559,165 oder US 5,527,536 beschrieben und zeichnen sich aus durch gute Klebeeigenschaften, einfache Herstellung und Verarbeitung sowie gute Hautverträglichkeit.

SxS Haftkleber können sowohl kommerziell bezogen werden (z.B. als Duro Tak 378-3500 bei National Starch & Chemical), als auch mit einem Heißschmelz Extrusions-Equipment selber bei der Produktion der wirkstoffhaltigen Pflaster hergestellt werden.

Dazu werden beispielsweise entsprechende Mengen (wenigstens folgender Bestandteile) eines Styrol-Block-Copolymers (z.B. Shell Kraton GX1657 oder Kraton D-1107CU) mit einem aliphatischen und/oder aromatischem Harz (z.B. Keyser Mackay Regalite R1090 oder Regalite R1010 oder Regalite R1100) und einem Öl (z.B. Shell Ondina 933 oder Ondina 941) aus den einzelnen Dosierstationen in den Extruder dosiert, dort vermischt und aufgeschmolzen. Im letzten Schritt wird in den so hergestellten Haftkleber der Wirkstoff in den Extruder eindosiert und die Masse auf Folien laminiert. Typische exemplarische Gewichtsanteile Polymer: Harz : Öl sind z.B. 100:120:20 oder 100:200:50. Durch Variation dieser Mengenanteile können die Eigenschaften des SxS-Haftklebers jeweils an die gewünschten Eigenschaften des TTS (Klebkraft, minimaler Kaltfluß, Klebezeitdauer, Freisetzungsprofil des Wirkstoffes, etc.) angepaßt werden.

Ein Gegenstand der Erfindung ist daher eine Vorrichtung zur transdermalen Verabreichung einer Verbindung der Formel I 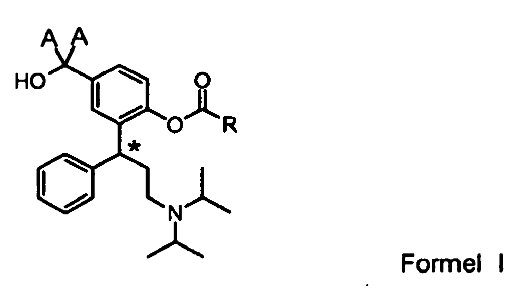 wobei A Wasserstoff oder Deuterium darstellt, R für eine Gruppe steht, die ausgewählt ist aus C1-6 Alkyl, C3-10 Cycloalkyl oder Phenyl, die jeweils mit C1-3 Alkoxy, Fluor, Chlor, Brom, Iod, Nitro, Amino, Hydroxy, Oxo, Mercapto oder Deuterium substituiert sein kann, wobei das mit "*" (Stern) markierte C-Atom in (R)-Konfiguration, in (S)-Konfiguration oder als Mischung davon vorliegen kann,
dadurch charakterisiert, dass die Verbindung der allgemeinen Formel I in Form der freien Base mit einem Salzgehalt von weniger als 10 Gew.-% in einer Reinheit von über 98 Gew.-%, bevorzugt über 97 Gew.-%, bevorzugt über 98 Gew.-%, besonders bevorzugt über 98,5 Gew.-% und ganz besonders bevorzugt über 99 Gew.-% in eine selbstklebende Polymerschicht (Klebermatrix) eingebracht wurde, wobei die Klebermatrix einen Haftkleber auf SXS-Basis umfasst.

In einer besonders bevorzugten Ausführungsform der Erfindung ist die SXS-basierte Klebermatrix im Heißschmelzverfahren hergestellt worden. Bevorzugt wurde in die erfindungsgemäße SXS-basierte Matrix als Wirkstoff die hochreine freie Base von Fesoterodin eingebracht.

Abbildungen 2 und 3 zeigen die in-vitro Fluxraten durch Humanhaut, bzw. Mäusehaut, die mit einem im Heißschmelzverfahren hergestellten SxS-basierten Pflaster, in das die hochreine freie Base von Fesoterodin eingebracht wurde, erreicht wurden.

Wegen der oxidativen Wirkung der SxS-Kleber, werden SxS-basierten Klebermatrices bevorzugt Antioxidanzien zugesetzt. Ein Beispiel für ein kommerziell erhältliches, geeignetes Antioxidanz ist Irganox^{R} (CIBA).

### Acrylatkleber:

Polyacrylate werden durch Radikal-Polymerisation von (Meth)Acrylsäure Derivaten produziert, wobei andere geeignete Verbindungen, wie z.B. Vinylacetat als weitere Monomere benutzt werden können. Der Ausdruck "Polyacrylat" umfasst in dieser Patentanmeldung Polymere, die Einheiten umfassen, die auf Acrylsäure und/oder Methacrylsäure beruhen sowie Copolymere und Mischungen davon.

Bei der Auswahl von geeigneten Monomeren können die daraus resultierenden Haftkleber prinzipiell so entworfen werden, dass sie spezifische Eigenschaften aufweisen, d.h. eine günstige Lösungskapazität für den Wirkstoff, eine gewünschte Beweglichkeit des Wirkstoffes in der Matrix sowie eine gewünschte Transfer-Rate über die Haut. Die Transfer-Rate wird wesentlich beschränkt durch den Distributions-Koeffizienten und die Resorption des Wirkstoffes durch die Haut.

Der drucksensitive Haftkleber des Polyacrylat-Typs kann ein Homopolymer und/oder Copolymer von mindestens einem Acrylsäure und/oder Meth-Acrylsäure Derivat in Form einer Lösung in einem organischen Lösungsmittel sein. Der Polyacrylat-Typ Haftkleber kann quervemetzbar oder nicht-quervernetzbar sein. Das quervernetzende Reagenz verbindet die Polymerketten mittels reaktiver Gruppen. Dies kann in erhöhter Cohäsion des Haftklebers resultieren.

Bevorzugt besteht der Polymer-Haftkleber des Polyacrylat-Typs mindestens aus den folgenden Monomeren:
Acrylsäure, Acrylamid, Hexyl-Acrylat, 2-Ethyl-Hexyl-Acrylat, Hydroxy-Ethyl-Acrylat, OctylAcrylat, Butyl-Acrylat, Methyl-Acrylat, Glycidyl-Acrylat, Methyl-Acrylat, Methacrylsäure, Methacrylamid, Hexyl-Methacrylat, 2-Ethyl-Hexylamid-Acrylat, Octyl-Methacrylat, MethylMethacrylat, Glycidyl-Methacrylat, Vinylacetat, Vinylpyrrolidon, Allyl-Acrylat.
Bevorzugt sind die Polymer Haftkleber des Acrylat-Typs quervernetzbare Haftkleber, die aus einer Kombination der folgenden Monomere polymerisiert werden:
   2-Ethyl-Hexyl-Acrylat/N-Butyl-Acrylat/Butyl-Acrylat/Acrylsäure,
   2-Ethyl-Hexyl-Acrylat/N-Butyl-Acrylat/Vinylacetat/Acrylsäure,
   2-Ethyl-Hexyl-Acrylat/Vinylacetat/Acrylsäure,
   2-Ethyl-Hexyl-Acrylat/Vinylacetat/Allyl-Acrylat,
   2-Ethyl-Hexyl-Acryla/Vinylacetat/Divinyl-Benzol/Acrylsäure,
   2-Ethyl-Hexyl-Acrylat/Vinylacetat/Allyl-Methacrylat/Acrylsäure,
   2-Ethyl-Hexyl-Acryla/Vinylacetat/2-Hydroxy-Ethyl-Acrylat,
   2-Ethyl-Hexyl-Acrylat/Vinylacetat/2-Hydroxy-Ethyl-Methacrylat,
   2-Ethyl-Hexyl-Acrylat/Fumarsäure-Diethyl-Ester/Acrylsäure,
   2-Ethyl-Hexyl-Acrylat/Maleinsäure-Diethyl-Ester/2-Hydroxy-Ethyl-Acrylat.

Als bevorzugte quervernetzbare Mittel können die folgenden Verbindungen genannt werden: Diphenyl-Methan-4-Diisocyanat, Hexamethylen-Diisocyanat, Titanium-Acetylacetonat, Aluminium-Acetylacetonat, Eisen-Acetylacetonat, Zink-Acetylacetonat, Magnesium-Acetylacetonat, Zirkonium-Acetylacetonat, 2-Ethyl-1,3-Hexanediol-Titanat, Tetra-Isooctyl-Titanat, Tetra-Nonyl-Titanat, polyfunktionale Propylen-Imin-Derivate, Ether-Derivate von Melamin-Formaldehyd-Harze, hoch methylierte Urethan-Harze, lmino-Melamin-Harze.

Die nicht-quervernetzbaren Haftkleber können bevorzugt aus einer Kombination der folgenden Monomere polymerisiert werden:
2-Ethyl-Hexyl-Acrylat/N-Butyl-Acrylat/Vinylacetat,
2-Ethyl-Hexyl-Acrylat/Vinylacetat,
2-Ethyl-Hexyl-Acrylat/N-Butyl-Acrylat/Vinylacetat/Allyl-Acrylat,
2-Ethyl-Hexyl-Acrylat/N-N-Butyl-Acrylat/Allyl-Methacrylat,
2-Ethyl-Hexyl-Acrylat/N-Butyl-Acrylat/Vinylacetat/Divinyl-Benzol,
2-Ethyl-Hexyl-Acrylat/Fumarsäure-Diethyl-Ester/Allyl-Acrylat,
2-Ethyl-Hexyl-Acrylat/Maleinsäure-Diethyl-Ester/Allyl-Acrylat,
2-Ethyl-Hexyl-Acrylat/N-Butyl-Acrylat/Acrylamid/Vinylacetat/Allyl-Acrylat,
2-Ethyl-Hexyl-Acrylat/N-Butyl-Acrylat/Iso-Butyl-Acrylat/Vinylacetat/Allyl-Acrylat.

Darüber hinaus können einige Haftkleber in Form wässriger Dispersion (Dispersionstyp) verwendet werden. Der Gebrauch dieser Dispersionstyp Haftkleber kann den Vorteil bringen, dass während des Überziehens und Trocknens keine brennbaren oder toxischen Lösungsmittel verdampfen.

Dispersionstyp Haftkleber können bevorzugt polymerisiert werden aus einer Kombination der folgenden Monomere:
N-Butyl-Acrylat/Iso-Butyl-Acrylat/Acrylsäure.
2-Ethyl-Hexyl-Acrylat/N-Butyl-Acrylat/Acesäure,
2-Ethyl-Hexyl-Acrylat/N-Buty-Acrylat/2-Hydroxy-Ethyl-Acrylamid,
2-Ethyl-Hexyl-Acrylat/N-Buty1-Acrylat/Vinylacetat/Acrylamid,
2-Ethyl-Hexyl-Acrylat/N-Butyl-Acrylat/Vinylacetat/2-Hydroxy-Ethyl-Acrylat,
2-Ethyl-Hexyl-Acrylat/N-Butyl-Acrylat/Allyl-Acrylat/Acrylsäure,
2-Ethyl-Hexyl-Acrylat/N-Butyl-Acrylat/Vinylacetat/Divinyl-Benzol.

Geeignete Polyacrylate zur Verwendung in der vorliegenden Erfindung werden durch mehrwertige Metall-Ionen quervernetzt, um die physikalischen Eigenschaften des Haftklebers zu verbessern oder um ihn an die spezifischen Anforderungen anzupassen. Die Metall-Ionen werden normalerweise in Form von Metall-Chelaten angewandt, welche in organischen Lösungsmitteln löslich sind. Besonders geeignete quervernetzende Mittel sind Aluminium-Acetyl-Acetonat und Titanium-Acetyl-Acetonat.

Falls der gemäß der vorliegenden Erfindung verwendete Haftkleber ein Polyacrylat-Haftkleber ist, hängt die Löslichkeitskapazität generell vom Typ und der Menge der freien funktionalen Gruppen in dem Haftkleber ab.

Die zur Verwendung in der Vorrichtung der vorliegenden Erfindung am meisten bevorzugten Haftkleber sind Polyacrylate mit polaren Gruppen, insbesondere mit freien Hydroxygruppen. Beispiele solcher Haftkleber sind Polyacrylate, zu deren Herstellung polare Monomere, wie z.B. Hydroxy-Ethyl-Acrylat, Hydroxy-Ethyl-Methacrylat, Acrylsäure oder Methacrylsäure in einer Menge von ca. 1-10 % (w/w), besonders bevorzugt in einer Menge von 3-8% (w/w), ganz besonders bevorzugt in einer Menge von 4-6 % (w/w) verwendet werden. Solche Haftkleber sind im Handel erhältlich unter dem Markennamen Duro-Tak^{®} (National Starch & Chemicals; Hamburg).

Ganz besonders bevorzugt zum Gebrauch in der Vorrichtung der vorliegenden Erfindung sind Haftkleber des Polyacrylat-Typs, wobei Hydroxy-Ethyl Acrylat und/oder Hydroxy-Ethyl Methacrylat Monomere während der Polymerisation in einer Menge von 3-8 % (w/w), ganz besonders bevorzugt in einer Menge von 4-6 % (w/w) beigemischt werden.

Solch ein Haftkleber kann entsprechend dem allgemeinen Verfahren erhalten werden, das im US Patent 5,498,418 wie folgt beschrieben wird: Der Haftkleber kann erhalten werden durch radikalische Polymerisation. Im ersten Schritt wird eine Mischung bestehend aus 21 bis 40 Gew.-% Vinylacetat, 55 bis 70 Gew.-% eines Acrylsäure-C₂₋₈-Alkyl-Esters und 3 bis 10 Gew.-% eines Acrylsäure-C₂₋₄-Hydroxyl-Acryl-Esters, mit 100 Gew.-% Monomeren in der Mischung, in einem organischen Lösungsmittel, hergestellt.

Im zweiten Schritt wird ein konventionelles quervemetzendes Mittel in einem organischen Lösungsmittel und - optional - der Wirkstoff in der für den beabsichtigten Gebrauch der transdermalen Vorrichtung (Pflaster) geforderten Qualität beigemischt, falls notwendig in einem organischen Lösungsmittel.

Schließlich wird in einem dritten Schritt die erhaltene Mischung des besonderen Arylat-Vinylacetat Copoloymers in einer zusätzlichen Stufe quervernetzt, begleitet durch Erhitzen und durch Entfernen des organischen Lösungsmittels oder der Mischung von verwendeten Lösungsmitteln. Der erhaltene Wirkstoff wird in die Haftklebersubstanz auf besondere Weise durch die aufeinanderfolgende und zusätzliche Quervernetzung des speziellen Acrylat-Vinylacetat Copolymers "eingebaut".

Alternativ kann das Acrylat-Vinylacetat-Copolymer in Abwesenheit des Wirkstoffes polymerisiert und quervernetzt werden. Der Wirkstoff wird dann erst während der Verwendung des Acrylat-Vinylacetat-Copolymers bei der Pflasterherstellung zugesetzt. Das Acrylat-Vinylacetat Copolymer hat eine relative Viskosität von 3,0 bis 4,2 bei 20° C.

Bevorzugt enthält die Mischung von Monomeren 2-Ethylhexylacrylat und Hyroxyethylacrylat zusätzlich zu Vinylacetat. Bevorzugt wird die anschließende Quervernetzung des speziellen Acrylat-Vinylacetat-Copolymers mit einem Titanium-Säure-Ester bestehend aus Polybutyl-Titantat und/oder Titanium-Acetylacetonat, durchgeführt, bevorzugt in einer Menge von 0,3 bis 3 Gew.-% im Verhältnis zum Gewicht des Copolymers.

Ein Prozess zur Herstellung eines TTS gemäß dieser Erfindung kann die folgenden Schritte umfassen: Als ersten Schritt Herstellung einer Lösung eines Copolymers, in welchem optional der Wirkstoff in der für den beabsichtigten Gebrauch des TTS erforderlichen Menge sowie ein konventioneller Quervernetzer oder eine Mischung davon enthalten ist, und wobei das Copolymer erhalten wird durch die radikale Polymerisation einer Mischung von Monomeren bestehend aus 21 bis 40 Gew.-% Vinylacetat, 55 bis 70 Gew.-% einer Acrylsäure-C₂₋₈-Alkyl-Ester und 1 bis 10 Gew.-% einer Acrylsäure-C₂₋₄-Hydroxyalkylester, Auftrag der oben genannten Lösung in der erforderlichen Schichtdicke auf dem Schutzfilm des TTS, und Entfernen des Lösungsmittels oder Mischung von Lösungsmitteln durch Erhitzen, was sich in einer zusätzlichen Quervernetzung des speziellen Acrylat-Vinylacetat Copolymers auswirkt.

Eine Ausführungsform solch eines Prozesses ist darin charakterisiert, dass das Acrylat-Vinylacetat-Copolymer, - optional - der Wirkstoff und das quervernetzbare Mittel anfangs in einem Lösungsmittel aufgelöst werden, welches 20 bis 40 Gew.-% Ethanol oder eine Ethanol-Methanol Mischung enthält, mit einem Verhältnis von festen Bestandteilen bestehend aus 40 bis 60 Gew.-% der Mischung des speziellen Acrylat-Vinylacetat Copolymers, des quervernetzbaren Mittels und des Wirkstoffes.

In einer anderen - bevorzugten - Ausführungsform der Erfindung wird der Wirkstoff erst nach Quervernetzung des Acrylats zur Dispersion gegeben, die dann nach Homogenisierung auf den Schutzfilm aufgetragen wird.

Ein besonderes Ausführungsbeispiel für die Zubereitung solch eines Acrylat-Vinylacetat Haftklebers wird offenbart in US-A-5,498,418, Spalte 2, Zeile 61 bis Spalte 3, Zeile 10, hier als Referenz angeführt.

Ein besonders bevorzugter Haftkleber zum Gebrauch in der vorliegenden Erfindung sind die im Handel erhältlichen Haftkleber Duro-Tak^{®} 387-2287 und Duro-Tak^{®} (3)87 4287 (National Starch & Chemicals; Hamburg). In einer besonders bevorzugten Ausführungsform der Erfindung wird der Duro-Tak-Haftkleber in geeignetem Lösungsmittel mit der gewünschten Menge des Wirkstoffes gemischt und die resultierende homogene Dispersion in gewünschter Dicke ausgestrichen. Schließlich wird das Lösungsmittel oder das Lösungsmittelgemisch bei erhöhten Temperaturen (50 - 70°C) entfernt.

Ein Gegenstand der Erfindung ist daher eine Vorrichtung zur transdermalen Verabreichung einer Verbindung der Formel I 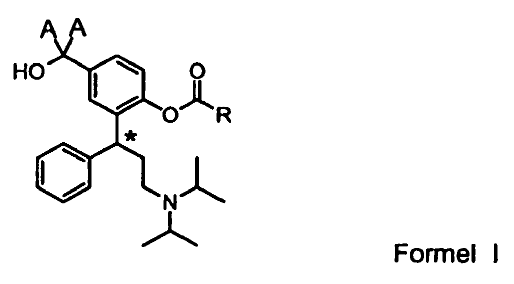 wobei A Wasserstoff oder Deuterium darstellt, R für eine Gruppe steht, die ausgewählt ist aus C1-6 Alkyl, C3-10 Cycloalkyl oder Phenyl, die jeweils mit C1-3 Alkoxy, Fluor, Chlor, Brom, Iod, Nitro, Amino, Hydroxy, Oxo, Mercapto oder Deuterium substituiert sein kann, wobei das mit "*" (Stern) markierte C-Atom in (R)-Konfiguration, in (S)-Konfiguration oder als Mischung davon vorliegen kann,
dadurch charakterisiert, dass die Verbindung der allgemeinen Formel I in Form der freien Base mit einem Salzgehalt von weniger als 10 Gew.-% in einer Reinheit von über 97 Gew.-%, bevorzugt über 98 Gew.%, besonders bevorzugt über 98,5 Gew.-% und ganz besonders bevorzugt über 99 Gew.-%, in eine Polymerschicht, vorzugsweise in eine selbstklebende Polymerschicht (Klebermatrix) eingebracht wurde, wobei die Polymerschicht mindestens ein Polymer vom Acrylat und/oder Methacrylat-Typ umfasst.

Bevorzugt wird in die erfindungsgemäße Acrylat-basierte Matrix als Wirkstoff die hochreine freie Base von Fesoterodin eingebracht.

Abbildung 3 zeigt die in-vitro Fluxrate durch Mäusehaut, die mit einem im Heißschmelzverfahren hergestellten Acrylat-basierten Pflaster, in das die hochreine freie Base von Fesoterodin eingebracht wurde, erreicht wurde.

### Hilfs- und Zusatzstoffe

Die oben beschriebenen wirkstoffhaltigen Polymermatrices der erfindungsgemäßen transdermalen Vorrichtungen können weitere Hilfs- und Zusatzstoffe enthalten. Beispiele sind Puffer, Lösungsvermittler, chemische Stabilisierungsmittel, Antioxidanzien, weitere Hilfsmittel zur Retardierung sowie Hautpenetrationsenhancer.

Hautpenetrationsenhancer können beispielsweise zugesetzt werden, um die Wirkstoffmenge, die durch die Haut permeiert zu vergrößern oder die Auftragsfläche der Vorrichtung zu verkleinern. Nicht-limitierende Beispiele für gängige Penetrationsenhancer sind Alkohole, insbesondere kurzkettige Alkohole wie Ethanol, Fettalkohole z.B. Laurylalkohol, Polyalkohole wie Glycerin; Amide, z.B. aromatische Amide wie N,N-Diethyl-m-Toluamid; Aminosäuren; Azone; Öle, wie z.B. Menthol oder Pfefferminzöl; Fettsäuren und deren Ester, wie z.B. Ölsäure, Laurylsäure, Isopropylmyristat oder Glycerolmonolaurat; Macrocylen, wie z.B. Cyclopentadecanon; Phospholipide wie z.B. Lecithin; 2-Pyrrolidone; Sulfoxide, wie z.B. Dimethylsulfoxid.

Auf Grund der guten Penetrationseigenschaften der freien Basen der allgemeinen Formel I werden Ausführungsformen der Erfindung bevorzugt, in denen auf die Zugabe eines Enhancers verzichtet wird.

Als weitere Bestandteile kann der Klebermatrix eine hydrophile Komponente, wie z.B. ein hydrophiles Polymer zugesetzt werden. Diese hydrophilen Polymere können als Löslichkeitsvermittler bzw. Kristallisationsinhibitoren für die Verbindungen der allgemeinen Formel I dienen und zu einer gleichmäßigen Verteilung des Wirkstoffs in der Klebermatrix beitragen.

Geeignete hydrophile Polymere zur Verwendung im erfindungsgemäßen TTS können beispielsweise ausgewählt sein aus der Gruppe der Polysaccharide, substituierte Polysaccharide, Polyethylenoxide, Polyvinylacetate, Polyvinylpyrrolidone (PVP), PVP mit geeignetem Weichmacher, Polyethylenglycole, Polypropylenglycole, Polyacrylate, Copolymere aus Polyvinylpyrrolidon und (Poly)vinylacetat, Copolymere von Ethylen und Vinylacetat sowie Polyvinylalkoholen mit geeignetem Weichmacher, z.B. Glycerin.

Bevorzugte hydrophile Polymere sind PVP, Polyethylenoxide (PEO), Polyvinylacetate (PVAc) sowie Copolymere aus PVP und Vinylacetat.

Die hydrophilen Polymere können der Kleberschicht beispielsweise in einem Anteil von 0,5-40 Gew-% bezogen auf das Gesamtgewicht der Kleberschicht zugesetzt werden. Bevorzugt werden 2-25 Gew-%, besonders bevorzugt 2-15 Gew-% oder 2-10 Gew-% hydrophile Polymere zugesetzt.

Zum Einsatz im Heißschmelzverfahren sind solche hydrophilen Polymere besonders geeignet, die bei Temperaturen unterhalb von 170°C eine dynamische Schmelzviskosität von maximal 150 Pa.s, bevorzugt kleiner als 120 Pa.s und besonders bevorzugt von unter 80 Pa.s aufweisen. Ist die dynamische Viskosität des hydrophilen Polymers bei der gewünschten Verarbeitungstemperatur zu gering, so muss gegebenenfalls ein geeigneter Weichmacher, z.B. Glycerin, vorab zugesetzt werden.

Der Zusatz der oben genannten hydrophilen Polymere kann insbesondere bei sehr hydrophoben Kleber-Matrices, z.B. Silikon-, Polyisobutylen- oder SXS-Matrices, vorteilhaft sein.

Wie bereits in der WO 01/35957 beschrieben, neigen die freien Basen der 3,3-Diphenylpropylamin-Monoester zur Gehaltsabnahme, z.B. durch Hydrolyse und Umesterung. Es wurde nun überraschend festgestellt, dass sich die 3,3-Diphenylpropylamin-Monoester in Matrices mit hydrophilen Anteilen signifikant stabilisieren lassen.

Während beispielsweise die freie Base von Fesoterodin als Öl nach 6-monatiger Lagerung bei 5°C zu ca. 3-4 % zersetzt ist, lässt sich eine Gehaltsabnahme nicht oder nur in wesentlich geringerem Maße feststellen, wenn Fesoterodin in Matrices eingearbeitet ist, die polare Bestandteile enthalten.

Beispiele für solche Matrices, die zur Stabilisierung des Monoesters der allgemeinen Formel 1 führen, sind beispielsweise Matrices, die Polyacrylate, insbesondere Polyacrylate mit polaren Gruppen, EVA oder Mischungen von Silikonklebern mit hydrophilen Polymeren, z.B. PVP oder PEO, enthalten (Tabelle 4).

**Tabelle 4: Stabilisierung von Fesoterodin in verschiedenen Matrices bei Lagerung**

| **Matrix** | **5°C** | **25°C / 60 %RH** | **Herstellverfahren** |
|---|---|---|---|
| | **Stabilislerungsfaktor¹** | **Stabilisierungsfaktor¹** | |
| | | | |
| | | | |
| EVA | 7-fach | 4,5-fach | Heißschmelz |
| Silikon/Cer³ | --- | --- | Heißschmelz |
| Silikon + 2 % PVP | 2-fach | 2-fach | Lösemittel |
| Silikon/Cer³ + 5 % PEO | 3-fach | 2,5-fach | Heißschmelz |
| | | | |
| Polyacrylat | kein Abbau nachweisbar² | 13-fach | Lösemittel |
| PIB | --- | --- | Lösemittel |
| SXS | --- | 1,1-fach | Heißschmelz |

| | | | |
|---|---|---|---|
| ¹Der Stabilisierungsfaktor wurde durch Division der durchschnittlichen monatlichen Gehaltsabnahme von Fesoterodin-Base bei Lagerung als Rohstoff (Öl) durch die durchschnittliche monatliche Gehaltsabnahme bei Lagerung in Matrices bestimmt; ² bis zum Ende der Meßperiode nach 6 Monaten; ³ Cer=Ceresin | | | |

Wie Tabelle 4 zeigt, führt die Einarbeitung von Fesoterodin in Matrices, bestehend aus EVA-Kleber, Polyacrylatkleber oder Mischungen aus Silikon-Kleber mit hydrophilen Polymeren wie PEO oder PVP zu einer deutlichen Stabilisierung von Fesoterodin, und zwar unabhängig vom Herstellverfahren (Heißschmelz- oder Lösemittelverfahren).

Eine Ausführungsform der Erfindung betrifft daher Matrices, bzw. pharmazeutische Formulierungen oder Vorrichtungen, in denen die Verbindungen der allgemeinen Formel I als freie Base einer langsameren Gehaltsabnahme unterworfen sind, als dies der Fall ist, wenn die freie Base unter identischen Bedingungen nicht in ein Polymer eingebettet als Öl gelagert wird. Bevorzugte Ausführungsformen sind solche, die bei 5°C und/oder bei 25°C zu wenigstens einer 2-, 3-, 7- oder 10-fachen Stabilisierung des 3,3-DiphenylpropylaminMonoesters im Vergleich zur Lagerung als freie Base führen.

Besonders bevorzugte erfindungsgemäße pharmazeutische Formulierungen bzw. Vorrichtungen sind solche, in denen die freie Base in einer Polymerschicht vorliegt, in der eine Gehaltsabnahme einer Verbindung der allgemeinen Formel I von weniger als 3 %, bevorzugt weniger als 2 % oder 1 % bei 6-monatiger Lagerung bei 4°C und von weniger als 10 %, bevorzugt weniger als 5 % und besonders bevorzugt weniger als 3 % oder 1,5 % bei 3-monatiger Lagerung bei 25°C und 60 % Luftfeuchtigkeit auftritt.

Bevorzugte Matrices sind solche, die 50-95 Gew-% eines Haftklebers enthalten, der ausgewählt ist aus der Gruppe der
- Acrylatkleber sowie deren Copolymere, insbesondere Acrylatkleber mit polaren Gruppen, z.B. mit freien Hydroxygruppen,
- EVA-Kleber
- Silikonkleber, die 2-25 Gew-%, bevorzugt 2-10 Gew-% eines hydrophilen Polymers, insbesondere ausgewählt aus PEO, PVP oder PVAc enthalten,
- SXS- oder PIB-Kleber, die 2-25 Gew-%, bevorzugt 2-10 Gew-% eines hydrophilen Polymers enthalten,
- Mischungen aus hydrophilen Haftklebern (z.B. polaren Polyacrylaten) mit hydrophoben Haftklebern (z.B. Silikon-, SXS- oder PIB-Klebern).

Ganz besonders bevorzugte Haftkleber zur Herstellung der erfindungsgemäßen Matrices sind Polyacrylate, insbesondere solche mit polaren Gruppen. Diese Matrices weisen sowohl ein exzellentes Freisetzungsprofil für Fesoterodin als auch hervorragende Stabilisierungseigenschaften für 3,3-Diphenylpropylamin-Monoester auf.

Als TTS-Größen werden von Patienten erfahrungsgemäß Grundflächen bis maximal ca. 50 cm² akzeptiert. Typischerweise hat das TTS eine Größe bis 40 cm², bevorzugt werden Größen zwischen 5 und 35 cm² und besonders bevorzugt zwischen 10 und 30 cm².

Das Matrixgewicht der TTS variiert typischerweise zwischen 30 und 300 g/m², wobei Matrices mit einem Gewicht von 40-200 g/m² und insbesondere 40-150 g/m² bevorzugt werden.

Die Beladung mit Wirkstoff hängt ab von der Aufnahme-/Freisetzungskapazität der jeweiligen Matrix für den Wirkstoff sowie vom Herstellungsverfahren.

Im allgemeinen ist eine Beladungsrate des Wirkstoffs zwischen 5 und ca. 40 Gew.-% bezogen auf das Gesamtgewicht der wirkstoffhaltigen Matrix sinnvoll, wobei niedrigere maximale Beladungsraten zwischen 7 und 30 Gew.-% und insbesondere zwischen 8 und 20 Gew.-% zur Herstellung von 1-3 Tages TTS bevorzugt werden. Soll ein Arzneimittel zur 7tägigen Verabreichung einer Verbindung der allgemeinen Formel I hergestellt werden, können vergleichsweise höhere Wirkstoffkonzentrationen von z.B. 15-14 Gew.-% eingesetzt werden.

Daraus resultiert eine Wirkstoffbeladung (mg/cm² Matrixfläche) von 0,1-12, bevorzugt 0,25-7,5, besonders bevorzugt von 0,3 bis 4 und ganz besonders bevorzugt von 0,6-2,5. Bei Vorrichtungen zur 7-tägigen Applikation liegt die Beladung bevorzugt bei mindestens 2 mg/m².

Ein weiterer Gegenstand der Erfindung ist eine Methode zur Vorbeugung und/oder zur Behandlung von Inkontinenz, Hyperaktivität des Detrusors, Hyperaktivität der Blase, Pollakisurie, Nykturie oder imperativem Harndrang durch die Verabreichung einer Verbindung der allgemeinen Formel I als freie Base und mit dem erfindungsgemäßen Reinheitsgrad, wie vorstehend beschrieben, an einen Säuger, insbesondere an einen Menschen, der der Vorbeugung oder Behandlung der vorgenannten Erkrankungen bedarf.

Die nachfolgenden Beispiele dienen der weiteren Illustration der Erfindung.

### Ausführungsbeispiele:

### 1. Herstellung der hochreinen freien Base von Fesoterodin

### A. Herstellung der Base Fesoterodin (B. s. Abbildung 1, R= i-Pr)

Zu einer auf -3° C gekühlten Lösung von 59,8 g (175,1 mol) (R)-2-[3-(Diisopropylamino)-1-phenylpropyl]-4-(hydroxymethyl)phenol (A, s. Abbildung 1) gelöst in 750 ml Dichlormethan wurde unter Rühren und Eisbadkühlung eine Lösung von 18,6 g Isobuttersäurechlorid in 250 ml Dichlormethan in ca. 10 Minuten zugetropft. Nach ca. 5 Minuten fiel weiße Substanz aus. Hierzu wurde unter Rühren und Eisbadkühlung eine Lösung von 17,7 g Triethylamin in 250 ml Dichlormethan in 5 Minuten zugetropft. Der Ansatz wurde nacheinander je einmal mit 250 ml Wasser, 250 ml ca. 5 %ige wässrige NaHCO₃ Lösung und 250 ml Wasser gewaschen. Der über Na₂SO₄ getrocknete Dichlormethanextrakt wurde am Rotationsverdampfer bis zur Gewichtskonstanz eingeengt, wobei ein blaßgelbes, zähflüssiges Öl übrigblieb.
Rohausbeute: 63,7g (88,5%).

Die Reinheit von B in der HPLC betrug in diesem Beispiel 94,1 %. Typischer Bereich für B: 90.5 % - 94.4 %: Beim Versuch der Hochvakuumdestillation trat unter Bildung von A und C Zersetzung ein.

### B. Herstellung des Fumaratsalzes (E; Abbildung 1 ; R = i-Pr, X = Hydrogenfumarat) von Fesoterodin

Eine Lösung von 41.87 g (102 mmol) (R)-2-(3-Diisopropylamino-1-phenylpropyl)-4-hydroxymethylphenylisobuttersäureester (B) in 90 ml 2-Butanon wurde unter Erwärmen mit Fumarsäure (11.81 g, 102 mmol) versetzt. Nach dem Lösen der Säure wurde langsam unter Rühren Cyclohexan (20-30 ml) bis zum Einsetzen einer Trübung zugesetzt. Man beließ den farblosen, homogenen Ansatz zunächst 18 Stunden bei Raumtemperatur, dann mehrere Stunden bei 0° C. Die ausgefallenen farblosen Kristalle wurden abgesaugt, mit wenig Cyclohexan/2-Butanon (90:10, Vol.-%) gewaschen und im Vakuum bei 30° C getrocknet.

Ausbeute: 44.6 g (83.1 % der Theorie) des Hydrogenfumarat-Salzes (E) des (R)-2-(3-Diisopropylamino-1-phenylpropyl)-4-hydroxymethylphenylisobuttersäureester in Form farbloser Plättchen.

Schmp. 98.8° C, eine zweite Kristallisation aus dem gleichen Lösungsmittelgemisch ergab das Produkt mit einem Schmp. von 103° C.

[α]_{D}²⁰ = + 6,0 (c = 1,0, Ethanol); - 19,3 (c = 1,0, Acetonitril).

¹H-NMR (CDCl₃): u.a. 6,84 ppm für C*H*= vom Hydrogenfumarat Anion.

¹³C-NMR (CDCl₃): u.a. 135,58 ppm und 170,56 ppm für Olefin- und Carbonylkohlenstoffe vom Hydrogenfumarat-Anion.

Die Reinheit in diesem Beispiel an E (bestimmt mit HPLC) betrug 99.2 %.

### C. Herstellung der hochreinen Base Fesoterodin (B: Abbildung 1: R= i-Pr),

250 g (0,474 mol) kristallines (R)-2-[3-(Diisopropylamino)-1-phenylpropyl]-4-(hydroxymethyl)-phenyl-2-methylpropanoat-Fumarsäuresalz (E) wurde unter Rühren zu 1 l Wasser gegeben und auf 30° C erwärmt. Nach ca. 30 Minuten lag eine fast klare Lösung vor. Zu der auf RT abgekühlten Lösung wurden unter Rühren in ca. 10 Minuten 96,0 g Natriumhydrogencarbonat portionsweise gegeben. Zur fast klaren, farblosen, wässrigen Lösung von Fesoterodin-Hydrogencarbonat wurde 1 l Dichlormethan gegeben. Nach einiger Zeit Rühren bei RT (starke CO₂-Entwicklung) wurde die Dichlormethanphase abgetrennt und nacheinander je einmal mit 0,2 Liter 5 %-iger wässriger Natriumhydrogencarbonatlösung und 0,2 l Wasser gewaschen. Die filtrierte klare, farblose Dichlormethanphase wurde am Rotavapor bei einer Badtemperatur von ca. 40° C bis zur Gewichtskonstanz eingeengt, wobei zuletzt Membranpumpenvakuum (Endvakuum 5 mbar) angelegt wurde. Dabei blieb (R)-2-(3-Diisopropylamino-1-phenylpropyl)-4-hydroxymethylphenylisobuttersäureester (B) als ein fast farbloses viskoses Öl übrig.
Ausbeute: 180,6 g (92,6%)
[α]_{D}²⁰ = + 5,9 (c = 1,0, Ethanol); - 6,7 (c = 1,0, Acetonitril)
NMR (CDCl₃): 19,01, 19,95, 20,59, 21,12, 34,28, 36,89, 41,88, 42,32, 43,90, 48,78, 64,68, 122,57, 125, 59, 126,16, 126,86, 127,96, 128,54, 136,88, 138,82, 143,92, 147,90, 175,69 (ppm).

Die Reinheit in der HPLC betrug in diesem Beispiel 99,0 %. Typische Reinheiten liegen zwischen 98,7% und 99,5%.
¹H- und ¹³C-NMR: Keine Resonanzsignale für das Hydrogenfumarat-Anion nachweisbar (vgl. E)

Die Langzeit-Lagerung erfolgt bevorzugt im Dunkeln unter Argon bei -20°C.

### D. Herstellung des Hydrogencarbonatsalzes (E; Abbildung 1; R = i-Pr, X = Hydrogencarbonat)

Fesoterodin (107,7 mg, (R)-2-(3-Diisopropylamino-1-phenylpropyl)-4-hydroxymethylphenylisobuttersäureester, B) wird mit destilliertem Wasser überschichtet und bei Raumtemperatur gerührt. Nach zwei Tagen Rühren bleibt der Reaktionsansatz unverändert zweiphasig. In der oberen, wässrigen Phase lässt sich dünnschichtchromatographisch kein organisches Material (B oder E) nachweisen (Kieselgel, Laufmittelsystem Petrolether/Aceton/Triethylamin, 70/20/10 Vol.-%).

In den zweiphasigen Reaktionsansatz wird bei Raumtemperatur unter Rühren ein leichter Strom Kohlendioxidgas eingeleitet. Nach zwei Tagen hat sich die untere ölige Phase (Fesoterodin) vollständig und klar in der wässrigen Phase gelöst.

¹³C-NMR-Spektrum des Hydrogencarbonatsalzes von Fesoterodin (δ-Werte):
14,11, 15,36, 15,51, 29,32, 31,09, 38,95, 43,31. 52,38, 60,45, 120,04, 124,07, 124,33, 124,83, 126,12, 131,97, 136.55, 139,06, 144,60, 157,46 (H*C*O₃⁻), 175,75.

Es ergibt sich eine gute Übereinstimmung mit dem ¹³C -NMR-Spektrum des Hydrochlorids von Fesoterodin, hergestellt durch Auflösen der Base in 1*M* wässriger Salzsäure (δ-Werte):
13,26, 15,32, 15,48, 29,29, 31.06, 38,95, 43,34, 52,42, 60,49, 120,10, 124,18, 124,38, 124,85, 126,13, 131,97, 136,50, 139,02, 144,61, 175,94.

### 2. Herstellung der TTS-Matrices

### 2.1. Herstellung einer silikonbasierten Matrix im Heißschmelzverfahren

8,5 g einer silikonbasierten Haftklebermischung aus dem Silikonkleber Bio-PSA 7-4300 (Dow.Corning, Michigan) mit 5 Gew.-% Ozokerit (bezogen bei Dow Corning) wurde für etwa 20 Minuten auf 150°C erhitzt bis eine homogene Schmelze entstand. 1.5 g Fesoterodine (hochreine freie Base) wurde zugesetzt und das Gemisch für weitere 5 Minuten bei 150°C gehalten. Die Mischung wurde dann manuell homogenisiert und auf eine vorgewärme Folie (120°C, Spaltweite 250 µm) laminiert. Für die Freisetzungstests wurden 5 cm² Stücke ausgeschnitten.

### 2.2. Herstellung einer acrylatbasierten Matrix im Lösemittelverfahren

1,5 g hochreine Fesoterodin-Base wurde in Dichlormethan gelöst und zu einer Lösung von 8,5 g DuroTak^{R} 387-2287 (in Ethylactetat) gegeben. Die resultierende Mischung wurde gerührt, bis eine homogene Dispersion erreicht wurde. Die Dispersion wurde dann auf Folie ausgetrichen (Erichsen 100µm, 6mm/sec, Trocknungszeit: 30 Min bei 50°C).

### 2.3. Herstellung eines SXS-basierten Matrix im Heißschmelzverfahren

100 Teile SIS (Kraton D1107CU), 150 Teile Regalite R 1090, 20 Teile Ondinaöl und 1 Teil Irganox wurden Hitze bei 140°C gemischt und aufgeschmolzen. Zu je 8,5 g Schmelze wurden 1.5 g Fesoterodin (hochreine freie Base) zugesetzt und das Gemisch für weitere 1-5 Minuten bei 140°C gehalten. Die Mischung wurde dann mechanisch homogenisiert und auf eine vorgewärme Folie (120°C, 250 µm) laminiert. Es wurden Stücke gewünschter Größe ausgeschnitten.

### 2.4. Herstellung einer EVA-basierten Matrix im Heißschmelzverfahren

8,5 g des EVA-Heißschmelzklebers wurden für etwa 20 Minuten bei 160°C erhitzt bis eine homogene Schmelze erhalten wurde. 1,5 g bzw. 1,65 g hochreine Fesoterodin-Base wurde dazugegeben und die Mischung sodann manuell homogenisiert. Die Mischung wurde dann auf eine vortemperierte (120°C) Chill-Roll laminiert. Es wurden jeweils 5 cm² (für Permeationsexperimente) ausgeschnitten.

### 3. Freisetzungsexperimente

### 3.1. Bestimmung des Wirkstoffflusses im Mäusehautmodell

Für die Fluxmessungen durch Mäusehaut wurde Bauch und Rückenhaut einer Dicke von ca. 120 bis 150 µm in einer horizontalen Diffusionszelle verwendet. Medium: PhosphatPufferlösung (0,066 molar) pH 6,2; 32°C

Die Wirkstoffreisetzung wurde per HPLC bestimmt.

### 3.2. Bestimmung des Wirkstofffluxes im Humanhautmodell

### (a) experimentelles Design

Die Bestimmung des Fesoterodinfluxes durch Humanhaut wurde im wesentlichen durchgeführt wie in H. Tanojo et al, J. Control Rel. 45 (1997) 41-47 beschrieben, wobei anstelle der Silikonmembran eine Dialysemembran [Diachema Dialysemembran, Typ 10.14, bezogen bei Fa. Dianorm, München, DE; hergestellt aus neutraler Zellulose, Ausschlußgröße 5000 Da, Dicke (trocken): 25 µm; Vorbehandlung gemäß Herstellerangaben] verwendet wurde.

Humanhaut wurde in einer Dicke von ca. 250 µm aus dem Abdomen gewonnen. Ein TTS mit einer Fläche von Fläche von 2,545 cm² wurde auf Humanhaut gleicher Fläche aufgebracht, wobei die Haut zur Akzeptorseite hin auf einer Silikonmembran aufliegt (Schema 1). Als Akzeptorphase wurde PBS (0,066 molar) bei PH 6,2 und einer Temperatur von 32±0.5°C verwendet. Die Experimente wurden mit einem Flux von 5mUh über 72 Stunden durchgeführt, wobei alle 3 Stunden Proben entnommen wurden. Zu den Probenentnahmezeiten wird das Freisetzungsmedium gegen frisches, auf 32±0.5°C thermostatiertes Medium ausgetauscht und die Menge des freigesetzten Fesoterodins per HPLC gemessen.

Die Bestimmung der Fluxrate Q(t) erfolgte bezogen auf die Fläche der Meßzelle (0.552 cm²) gemäß der Formel:
Q(t) = µg/cm² = Konzentration Fesoterodin Volumen des Akzeptors/0.552 cm²

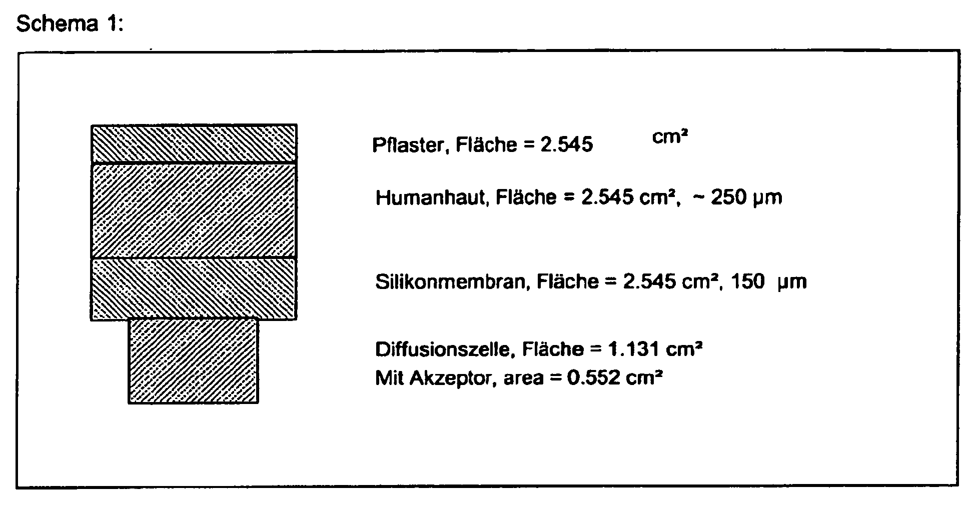

### (b) Analytik der Wirkstofffreisetzung

Die Messung des Wirkstofffluxes durch Hautpräparate erfolgte per HPLC (Säule Spherisorb 5CN 25cm) unter folgenden Bedingungen: 4 Volumenteile Acetonitril / 6 Volumenteile H₂O / 0,1% Volumenteile TFA; 35°C, 225 nm, 1ml Fluss

### 4. Analytik: Bestimmung der Reinheit des Wirkstoffs

Zur Bestimmung der chemischen Reinheit von Fesoterodin wurde eine HPLC-Methode verwendet, die auf der Trennung an einer stationären Umkehrphase (reversed phase) beruht und zur Elution einen Lösungsmittelgradienten verwendet.

### Materialien: (beispielhaft):

Acetonitril für die HPLC, Methansulfonsäure (<99%, Fluka), Wasser (Gereinigt, HPLC-Qualität), Waters Pumpe 510, Säulenofen (Waters Column Heater Modul, 35 °C), Probengeber (Waters Wisp 717, Injektionsvolumen 20 µL), UV-Detektor (Shimatzu SPD 10A). Säule (150x3,9 mm, Symmetry Shield RP8, Waters Part No. WAT 200655).

### Mobile Phase:

Acetonitril mit 0,05% Methansulfonsäure (v/v, %), Komponente A
Wasser mit 0,05% Methansulfonsäure (v/v, %), Komponente B
Gradientenprogramm: Zeit (Min.) 0,0 mit 15% Komponente A und 85% Komponente B, nach 15 Min. 60% A und 40% B, nach 20 Min. 15% A und 85% B. Flußrate: 1,2 ml/Min.

Die Konzentrationen der Referenzlösungen von A, B und C (Abbildung 1/4, R = i-Pr) betrugen 10-250 µg/mL. Bei höheren Konzentrationen trat Tailing mit Peaküberlappung auf.

### Auswertung:

Zur Auswertung nach der 100%-Methode wurden die Mittelwerte aller Peakflächen (Dreifachbestimmungen) addiert und gleich 100% gesetzt. Auf diesen Wert wurden die Flächen der einzelnen Peaks (in %) bezogen. Retentionszeiten für A, B und C (Min.): 5,9, 9,0 und 12,6.

### 5. Analytik: Bestimmung des Restsalzgehaltes

Es werden 200 MHz oder 500 MHz ¹H-NMR-Spektren der freien Base Fesoterodin in CDCl₃ als Lösungsmittel aufgenommen und charakteristische Resonanzsignal-Gruppen elektronisch integriert, wie:
δ = 6,97 ppm (Duplett, aromatischer Wasserstoff, H⁶, 1H),
δ = 4,59 ppm (Singulett, HO-C*H*₂, 2H),
8 = 4,10 ppm (Triplett, H¹-Propyl, 1H).

Die Relation zum Resonanzsignal des Anions, z.B. Hydrogenfumarat
(δ = 6.84 ppm, =C*H*-, 2H) ergibt den Anteil an Restsalz (in Mol-%).

## Patentansprüche

1. Pharmazeutische Formulierung enthaltend eine Verbindung der allgemeinen Formel I, 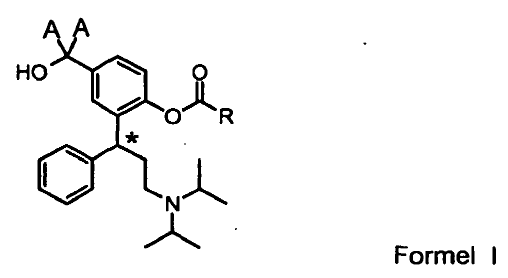 wobei A Deuterium oder Wasserstoff ist, R für eine Gruppe steht, die ausgewählt ist aus C₁₋₆ Alkyl, C₃₋₁₀ Cycloalkyl oder Phenyl, die jeweils mit C₁₋₃ Alkoxy, Fluor, Clor, Brom, lod, Nitro, Amino, Hydroxy, Oxo, Mercapto oder Deuterium substituiert sein kann und wobei das mit "*" (Stern) markierte C-Atom in (R)-Konfiguration, in (S)-Konfiguration oder als Mischung davon vorliegen kann,
und einen pharmazeutisch akzeptablen Träger,
**dadurch** charakterisiert, dass die besagte Verbindung als freie Base mit einem Salzgehalt von weniger als 10 Gew% und einem Reinheitsgrad von über 97 Gew% vorliegt.

2. Pharmazeutische Formulierung nach Anspruch 1, wobei R ausgewählt ist aus der Gruppe Methyl, Ethyl, iso-Propyl, 1-Propyl, 1-Butyl, 2-Butyl, tert.-Butyl, iso-Butyl, Pentyl und Hexyl.

3. Pharmazeutische Formulierung nach einem der vorhergehenden Ansprüche, wobei die Verbindung 2-[3-(1,1 -Diisopropylamino)-1-phenylpropyl]-4-(hydroxymethyl)phenyl isobutyrat ist.

4. Pharmazeutische Formulierung nach einem der vorhergehenden Ansprüche, **dadurch gekennzeichnet, dass** das mit "*" markierte C-Atom in (R)-Konfiguration vorliegt.

5. Pharmazeutische Formulierung nach einem der vorhergehenden Ansprüche, wobei die Verbindung (R) 2-[3-(1,1-Diisopropylamino)-1-phenylpropyl]-4-(hydroxymethyl)phenyl isobutyrat (Fesoterodine) ist.

6. Pharmazeutische Formulierung nach einem der vorhergehenden Ansprüche, wobei der pharmazeutisch akzeptable Träger ein Polymer ist.

7. Pharmazeutische Formulierung nach einem der vorhergehenden Ansprüche, **gekennzeichnet durch** die Stabilisierung der Verbindung der Formel I in der pharmazeutischen Formulierung, wobei der Stabilisierungsfaktor; erhalten **durch** Division der durchschnittlichen monatlichen Gehaltsabnahme der Verbindung der Formel 1 bei Lagerung als Öl und in Abwesenheit des pharmazeutisch akzeptablen Trägers bei 5°C durch die durchschnittliche monatliche Gehaltsabnahme der entsprechenden Verbindung der Formel 1 bei Lagerung in besagter pharmazeutischer Formulierung bei 5°C, mindestens 2 ist.

8. Pharmazeutische Formulierung nach einem der Ansprüche 1-7, wobei die Formulierung einen pH-Wert von 3,0-6,0 aufweist

9. Pharmazeutische Formulierung nach einem der vorhergehenden Ansprüche, wobei die pharmazeutische Formulierung zur transdermalen oder transmukosalen Verabreichung geeignet ist.

10. Pharmazeutische Formulierung nach einem der vorhergehenden Ansprüche, wobei die pharmazeutische Formulierung eine Polymerschicht enthält, in der eine Verbindung der allgemeinen Formel I, wie in einem der Ansprüche 1-5 definiert, gelöst oder dispergiert ist.

11. Pharmazeutische Formulierung nach Anspruch 10, wobei die Polymerschicht einen Haftkleber enthält, der die Fixierung der pharmazeutischen Formulierung auf der Haut oder Schleimhaut des Patienten ermöglicht.

12. Pharmazeutische Formulierung nach Anspruch 11, wobei die Polymerschicht einen Haftkleber enthält, der die Fixierung der pharmazeutischen Formulierung auf der Haut des Patienten ermöglicht und der ausgewählt ist aus der Gruppe der Silikon-, Acrylat-, SXS-, PIB- oder EVA-basierten Haftkleber.

13. Pharmazeutische Formulierung nach einem der vorhergehenden Ansprüche, wobei die pharmazeutische Formulierung ein Transdermales Therapeutisches System vom Wirkstoff-in-Kleber Typ ist.

14. Pharmazeutische Formulierung nach einem der vorhergehenden Ansprüche, ausgebildet als Dosierungseinheit, die mindestens 3 mg einer Verbindung der allgemeinen Formel 1, wie in einem der Ansprüche 1-5 definiert, enthält.

15. Pharmazeutische Formulierung nach Anspruch 14, wobei die Verbindung (R) 2-[3-(1,1-Diisopropylamino)-1-phenylpropyl]-4-(hydroxymethyl)phenyl isobutyrat (Fesoterodine) ist.

16. Kit, enthaltend eine pharmazeutische Formulierung nach einem der vorhergehenden Ansprüche und ein Trocknungsmittel

17. Herstellung einer pharmazeutischen Formulierung nach einem der Ansprüche 1-5, umfassend die Herstellung einer Verbindung der allgemeinen Formel 1 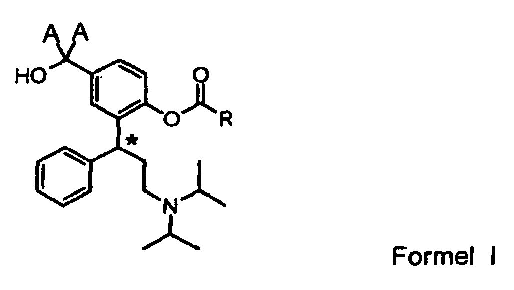 wobei A für Deuterium oder Wasserstoff, R für eine Gruppe steht, die ausgewählt ist aus C₁₋₆ Alkyl, C₃₋₁₀ Cycloalkyl oder Phenyl steht, die jeweils mit C₁₋₃ Alkoxy, Fluor, Clor, Brom, Iod, Nitro, Amino, Hydroxy, Oxo, Mercapto oder Deuterium substituiert sein kann und wobei das mit "*" (Stern) markierte C-Atom in (R)-Konfiguration, in (S)-Konfiguration oder als Mischung davon vorliegen kann, als freie Base in einer Reinheit von wenigstens 97 Gew%,
durch Freisetzung der Base aus einem kristallinen Salz der allgemeinen Formel II 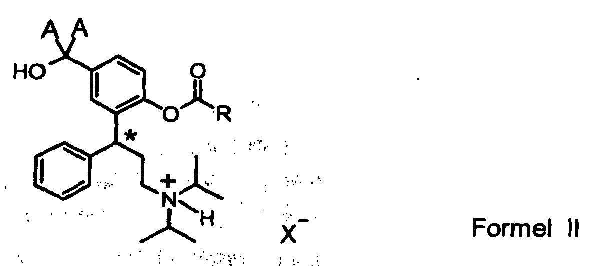 mit einem Reinheitsgrad von wenigstens 97 Gew%, wobei A und R die oben angegebene Bedeutung haben und X⁻ der Säurerest einer physiologisch verträglichen Säure ist, und wobei das mit "*" (Stem) markierte C-Atom in (R)-Konfiguration, in (S)-Konfiguration oder als Mischung davon vorliegen kann,
**dadurch gekennzeichnet, dass** die Umsetzung der Verbindung der Formel II mit einem Freisetzungsreagenz in wässriger Lösung erfolgt, wobei das Freisetzungsreagenz einen pK_{B} von 8 - 11 hat, nicht zur Präzipitation der Verbindungen der Formel I führt, und das ausgewählt ist aus der Gruppe
(a) der Alkali-, Erdalkali- oder Ammonium-Hydrogencarbonate
(b) der Amine, Polyamine und basischen Polyaminosäuren und
(c) der basischen Ionentauscher,
und das Mischen der so enthaltenen Verbindung mit einem pharmazeutisch akzeptablen Träger.

18. Herstellungsverfahren nach Anspruch 17, **dadurch gekennzeichnet, dass** die Verbindung der Formel 1 aus einem kristallinen Salz der Formel 11 durch Zugabe eines Alkali-, Erdalkali- oder Ammonium-Hydrogencarbonats freigesetzt wird.

19. Herstellungsverfahren nach einem der vorhergehenden Ansprüche, **dadurch gekennzeichnet, dass** nach der Freisetzung der hochreinen Base der Formel I aus dem Salz der Formel II die wässrige Lösung mit einem Lösemittel ausgeschüttelt wird und die hochreine Base der allgemeinen Formel I sodann durch Einengen der organischen Phase gewonnen wird, wobei das Lösemittel ausgewählt ist aus der Gruppe Dichlormethan, Ethylmethylketon, Ethylacetat, tertiärer Butylmethylether, Diethylether sowie Toluol.

20. Herstellungsverfahren nach einem der vorhergehenden Ansprüche, **dadurch gekennzeichnet, dass** R ausgewählt ist aus der Gruppe Methyl, Ethyl, iso-Propyl, 1-Propyl, 1-Butyl, 2-Butyl, tert.-Butyl-, iso-Butyl, Pentyl und Hexyl und wobei das mit "*" (Stern) **gekennzeichnet**e C-Atom in (R)-Konfiguration vorliegt.

21. Herstellungsverfahren nach einem der vorhergehenden Ansprüche, wobei die Verbindung der Formel I (R)-2-[3-(1,1-Diisopropylamino)-1-phenylpropyl]-4-(hydroxymethyl)phenyl isobutyrat ist.

22. Herstellungsverfahren nach einem der vorhergehenden Ansprüche, wobei die Verbindung der Formel II (R)-2-[3-(1,1-Diisopropylammonio)-1-phenylpropyl]-4-(hydroxymethyl)phenyl isobutyrat hydrogenfumarat ist.

23. Fesoterodin Hydrogencarbonat.

## Claims

1. Pharmaceutical formulation comprising a compound of the general Formula I, 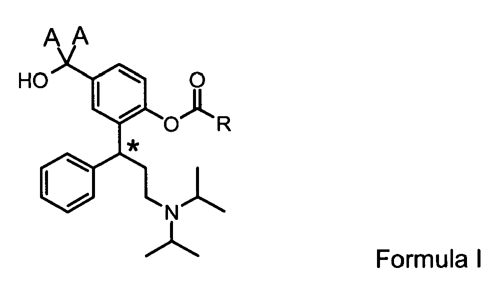 in which A means deuterium or hydrogen, R stands for a group that is selected from C₁₋₆-alkyl, C₃₋₁₀-cycloalkyl or phenyl, which may each be substituted with C₁₋₃-alkoxy, fluorine, chlorine, bromine, iodine, nitro, amino, hydroxyl, oxo, mercapto or deuterium and where the C-atom marked with a star "*" may be present in the (R)-configuration, the (S)-configuration or as a mixture of it,
and a pharmaceutically active carrier,
**characterized by** the fact that the said compound is present as a free base with a salt content of below 10wt% and in a degree of purity of above 97 percent by weight.

2. Pharmaceutical formulation according to claim 1, whereby R is selected from the group methyl, ethyl, isopropyl 1, 1-propyl, 1-butyl, 2-butyl, tertiary-butyl, iso-butyl, pentyl and hexyl.

3. Pharmaceutical formulation according to one of the previous claims, whereby the compound is 2-[3-(1,1-diisopropylamino)-1-phenylpropyl]-4-(hydroxymethyl)phenyl isobutyrate.

4. Pharmaceutical formulation according to one of the previous claims, **characterized by** that the C-atom marked with "*" is present in the (R)-configuration.

5. Pharmaceutical formulation according to one of the previous claims, whereby the compound is (R)-2-[3-(1,1-diisopropylamino)-1-phenylpropyl]-4-(hydroxymethyl)phenyl isobutyrate (fesoterodine).

6. A pharmaceutical formulation according to one of the previous claims, whereby the pharmaceutically acceptable carrier is a polymer.

7. A pharmaceutical formulation according to one of the previous claims **characterized by** the stabilization of the compound of the Formula I in the pharmaceutical formulation, whereby the stabilization factor, determined by the division of the average monthly drop in concentration of the compound of Formula I during storage as oil and in the absence of the pharmaceutically acceptable carrier at 5°C by the average monthly drop in concentration of the corresponding compound of Formula I during storage in the said pharmaceutical formulation at 5°C, is at least 2.

8. A pharmaceutical formulation according to anyone of claims 1-7, whereby the formulation exhibits a pH value of 3.0-6.0.

9. A pharmaceutical formulation according to one of the previous claims, whereby the pharmaceutical formulation is suitable for transdermal or transmucosal delivery.

10. A pharmaceutical formulation according to one of the previous claims, whereby the pharmaceutical formulation contains a polymer layer, in which a compound of the general Formula I, as defined in one of the claims 1-5, is either dissolved or dispersed.

11. A pharmaceutical formulation according to claim 10, whereby the polymer layer contains a contact adhesive, which makes the attachment of the pharmaceutical composition to the skin or the mucous membrane of the patient possible.

12. A pharmaceutical formulation according to claim 11, whereby the polymer layer contains a contact adhesive, which makes the attachment of the pharmaceutical composition to the skin of the patient possible and which is chosen from the group of silicone, acrylate, SXS-, PIB- or EVA based contact adhesives.

13. A pharmaceutical formulation according to one of the previous claims, whereby the pharmaceutical formulation is a transdermal therapeutic system of the active ingredient-in-adhesive type.

14. A pharmaceutical formulation according to one of the previous claims, and being a dosing unit, which contains at least 3 mg of a compound of the general Formula I, as defined in one of the claims 1-5.

15. A pharmaceutical formulation according to claim 14, whereby the compound is (R) 2-[3-(1,1-Diisopropylamino)-1-phenylpropyl]-4-(hydroxymethyl)phenyl isobutyrate (fesoterodine).

16. A kit containing a pharmaceutical formulation according to one of the previous claims, and a drying agent.

17. Manufacture of a pharmaceutical formulation according to claims 1-5 comprising the manufacture of a compound of the general Formula I as a free base in a purity of at least 97 percent by weight, 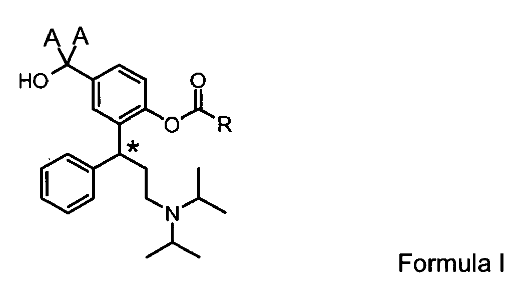 wherein A means hydrogen or deuterium, R stands for a group, which is selected from C₁₋₆-alkyl, C₃₋₁₀-cycloalkyl or phenyl, which may each be substituted with C₁₋₃-alkoxy, fluorine, chlorine, bromine, iodine, nitro, amino, hydroxyl, oxo, mercapto or deuterium, and wherein the C-atom marked with a star "*" may be present in the (R)-configuration, in the (S)-configuration or as a mixture of it,
through the release of the base from a crystalline salt of the general Formula II 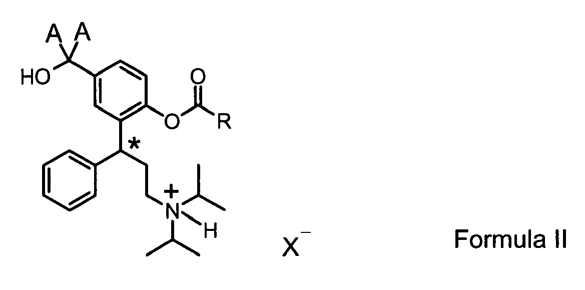 with a degree of purity of at least 97 percent by weight where in A and R have the meaning given above and X- is the acid residue of a physiological compatible acid and wherein the C-atom marked with "*" (a star) can be present in the (R)-configuration, in the (S)-configuration or as a mixture thereof, **characterized by** that the conversion of the compound of Formula II is done using a releasing reagent in aqueous solution, whereby the releasing reagent has a pK_{B} of 8 - 11 and does not lead to the precipitation of the compounds of Formula I, and is chosen from the group of
(a) the alkaline, alkaline earth- or ammonium hydrogen carbonates
(b) the amines, polyamines and alkaline polyamino acids and
(c) the alkaline ionic exchangers,
and
the mixture of the thus obtained compound with a pharmaceutically active carrier.

18. A manufacturing procedure according to claim 17, **characterized by** the fact that the free base of the general Formula I is released from the crystalline salt of the general Formula II through the addition of an alkaline, earth-alkaline or ammonium hydrogen carbonate.

19. A manufacturing procedure according to one of the previous claims, **characterized by** the fact that after the release of the high purity base of the Formula I from the salt of the Formula II, the aqueous solution is extracted by shaking with an organic solvent selected from the group of dichlormethane, ethyl methyl ketone, ethyl acetate, tertiary butyl methyl ether, diethylether as well as toluene, and the high purity base of the general Formula I is then yielded by the organic phase being concentrated to a small volume.

20. A manufacturing procedure according to one of the previous claims, **characterized by** the fact that the R is selected from the group methyl, ethyl, isopropyl, 1-propyl, 1-butyl, 2-butyl, tertiary butyl, iso-butyl, pentyl and hexyl and whereby the C-atom marked with an "*" (star) is present in the (R)-configuration.

21. A manufacturing procedure according to one of the previous claims, whereby the compound of the Formula I is (R)-2-[3-(1,1-diisopropylamino)-1-phenylpropyl]-4-(hydroxymethyl)phenyl isobutyrate.

22. A manufacturing procedure according to one of the previous claims, whereby the compound of the Formula II is (R)-2-[3-(1,1-diisopropylamino)-1-phenylpropyl]-4-(hydroxymethyl)phenyl isobutyrate hydrogen fumarate.

23. Fesoterodine hydrogen carbonate.

## Revendications

1. Formulation pharmaceutique contenant un composé selon la formule générale I, 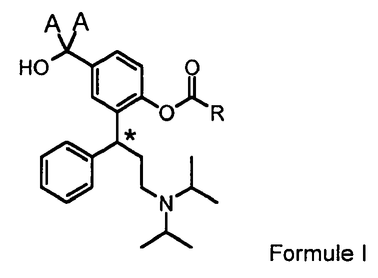 dans laquelle A est le deutérium ou l'hydrogène, R représente un groupe choisi parmi alkyle en C₁-C₆, cycloalkyle en C₃-C₁₀ ou phényle, chacun pouvant être substitué avec alkoxy en C₁-C₃, fluor, chlore, brome, iode, nitro, amino, hydroxy, oxo, mercapto ou deutérium et dans laquelle l'atome de carbone marqué du "*" (astérisque) se présente dans la configuration (R), la configuration (S) ou une configuration mixte,
et un véhicule pharmaceutiquement acceptable,
***caractérisé en ce que*** ledit composé est présent sous la forme de la base libre avec une teneur en sel inférieure à 10 % en poids et une pureté supérieure à 97 % en poids.

2. Formulation pharmaceutique selon la revendication 1, dans laquelle R est choisi parmi méthyle, éthyle, isopropyle, 1-propyle, 1-butyle, 2-butyle, tert-butyle, iso-butyle, pentyle et hexyle.

3. Formulation pharmaceutique l'une des revendications précédentes, dans laquelle le composé est l'isobutyrate de 2-[3-(1,1-di-isopropylamino)-1-phénylpropyl]-4-(hydroxyméthyl)phényle.

4. Formulation pharmaceutique l'une des revendications précédentes, dans laquelle l'atome de carbone marqué du "*" se présente dans la configuration (R).

5. Formulation pharmaceutique l'une des revendications précédentes, dans laquelle le composé (R) est l'isobutyrate de 2-[3-(1,1-di-isopropylamino)-1-phénylpropyl]-4-(hydroxyméthyl)phényle (fésotérodine).

6. Formulation pharmaceutique l'une des revendications précédentes, dans laquelle le véhicule pharmaceutiquement acceptable est un polymère.

7. Formulation pharmaceutique l'une quelconque des revendications précédentes, ***caractérisée en ce que*** le composé selon la formule 1 est stabilisé dans la formulation pharmaceutique, le facteur de stabilisation, obtenu par division de l'appauvrissement mensuel moyen du composé selon la formule I pendant un stockage sous forme d'huile et en l'absence du véhicule pharmaceutiquement acceptable à 5°C par l'appauvrissement mensuel moyen du composé selon la formule I pendant un stockage dans ladite formulation pharmaceutique à 5°C, étant d'au moins 2.

8. Formulation pharmaceutique l'une quelconque des revendications 1 à 7, laquelle la formulation a un pH de 3,0 à 6,0.

9. Formulation pharmaceutique l'une quelconque des revendications précédentes, laquelle formulation pharmaceutique convient pour une administration transdermique ou transmuqueuse.

10. Formulation pharmaceutique l'une quelconque des revendications précédentes, laquelle formulation pharmaceutique contient une couche de polymère dans laquelle un composé selon la formule générale I, tel qu'il est défini dans l'une quelconque des revendications 1 à 5, est dissous ou dispersé.

11. Formulation pharmaceutique selon la revendication 10, dans laquelle la couche de polymère contient un adhésif permettant de fixer la formulation pharmaceutique sur la peau ou la muqueuse du patient.

12. Formulation pharmaceutique selon la revendication 11, dans laquelle la couche de polymère contient un adhésif permettant de fixer la formulation pharmaceutique sur la peau ou la muqueuse du patient et choisi dans le groupe constitué par les adhésifs à base de silicone, d'acrylate, de SXS, de PIB ou d'EVA.

13. Formulation pharmaceutique l'une quelconque des revendications précédentes, dans laquelle la formulation pharmaceutique est un système thérapeutique transdermique dans lequel le principe actif est contenu dans l'adhésif.

14. Formulation pharmaceutique l'une quelconque des revendications précédentes, conçue comme une unité d'administration contenant au moins 3 mg d'un composé selon la formule générale I, tel qu'il est défini dans l'une quelconque des revendications 1 à 5.

15. Formulation pharmaceutique selon la revendication 14, dans laquelle le composé (R) est l'isobutyrate de 2-[3-(1,1-di-isopropylamino)-1-phénylpropyl]-4-(hydroxyméthyl) phényle (fésotérodine).

16. Kit contenant une formulation pharmaceutique selon l'une quelconque des revendications précédentes et un agent dessiccateur.

17. Fabrication d'une formulation pharmaceutique selon l'une quelconque des revendications 1 à 5,
comprenant la fabrication d'un composé selon la formule générale 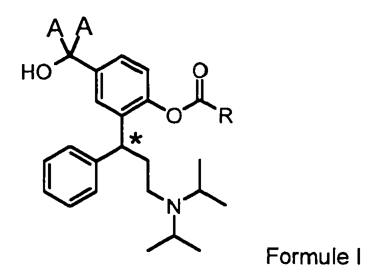 dans laquelle A est le deutérium ou l'hydrogène, R représente un groupe choisi parmi alkyle en C₁-C₆, cycloalkyle en C₃-C₁₀ ou phényle, chacun pouvant être substitué avec alkoxy en C₁-C₃, fluor, chlore, brome, iode, nitro, amino, hydroxy, oxo, mercapto ou deutérium et dans laquelle l'atome de carbone marqué du "*" (astérisque) se présente dans la configuration (R), la configuration (S) ou une configuration mixte, sous forme de base libre avec une pureté d'au moins 97 % en poids,
par libération de la base à partir d'un sel cristallin selon la formule générale II 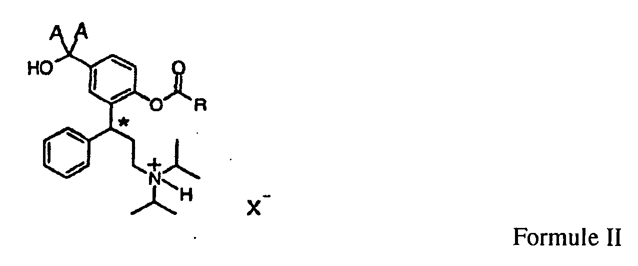 avec une pureté d'au moins 97 % en poids, A et R ayant la signification indiquée ci-dessus et X- étant le résidu acide d'un acide physiologiquement compatible, et l'atome de carbone marqué du "*" (astérisque) se présentant dans la configuration (R), la configuration (S) ou une configuration mixte,
***caractérisée en ce que*** la transformation du composé selon la formule II est réalisée avec un réactif de libération dans une solution aqueuse, le réactif de libération ayant un pK_{B} de 8 à 11, ne provoquant pas la précipitation des composés selon la formule I et étant choisi dans le groupe constitué par
(a) les hydrogénocarbonates de métaux alcalins, alcalino-terreux ou d'ammonium
(b) les amines, polyamines et acides polyaminés basiques, et
(c) les échangeurs d'ions basiques,
et le composé ainsi obtenu est mélangé avec un véhicule pharmaceutiquement acceptable.

18. Procédé de fabrication selon la revendication 17, ***caractérisé en ce que*** le composé selon la formule 1 à partir d'un sel cristallin selon la formule II est libéré par addition d'un hydrogénocarbonate de métal alcalin, alcalino-terreux ou d'ammonium.

19. Procédé de fabrication selon l'une quelconque des revendications précédentes, **ca*ractérisé en ce qu'***après la libération de la base ultra-pure selon la formule 1 à partir du sel selon la formule II, la solution aqueuse est agitée avec un solvant et la base ultra-pure selon la formule générale I est ensuite obtenue par réduction de la phase organique, le solvant étant choisi dans le groupe constitué par le dichlorométhane, l'éthylméthylcétone, l'acétate d'éthyle, l'éther de butyle tertiaire-méthyle, l'éther diéthylique et le toluène.

20. Procédé de fabrication selon l'une quelconque des revendications précédentes, ***caractérisé en ce que*** R est choisi dans le groupe constitué par le méthyle, l'éthyle, l'isopropyle, le 1-propyle, le 1-butyle, le 2-butyle, le tert-butyle-, l'isobutyle, le pentyle et l'hexyle et dans lequel l'atome de carbone marqué du "*" (astérisque) se présente dans la configuration (R).

21. Procédé de fabrication selon l'une quelconque des revendications précédentes, dans lequel le composé selon la formule I est le (R)-isobutyrate de 2-[3-(1,1-di-isopropylamino)-1-phénylpropyl]-4-(hydroxyméthyl)phényle.

22. Procédé de fabrication selon l'une quelconque des revendications précédentes, dans lequel le composé selon la formule II est l'isobutyrate-hydrogénofumarate de (R)-2-[3-(1,1-di-isopropylammonio)-1-phénylpropyl]-4-(hydroxyméthyl)phényle.

23. Hydrogénocarbonate de fésotérodine.
